# EUROPÄISCHE PATENTSCHRIFT

(11) **EP 1 711 480 B1**
(45) Veröffentlichungstag und Bekanntmachung des Hinweises auf die Patenterteilung: **19.12.2007**
(21) Anmeldenummer: 05700863.3
(22) Anmeldetag: 13.01.2005
(51) Int. Cl.: C07D 265/36, C07D 413/12, A61K 31/538, A61P 11/08

(54) **NEUE LANG WIRKSAME BETA-2-AGONISTEN, UND DEREN VERWENDUNG ALS ARZNEIMITTEL**
NOVEL LONG-WORKING BETA-2-AGONISTS AND USE THEREOF AS MEDICAMENTS
NOUVEAUX BETA-2 AGONISTES A EFFET PROLONGE ET LEUR UTILISATION COMME MEDICAMENTS

(30) Priorität: 23.01.2004 DE 102004003428
(43) Veröffentlichungstag der Anmeldung: 18.10.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: HOENKE, Christoph, 55218 Ingelheim (DE); RUDOLF, Klaus, 88447 Warthausen (DE); BOUYSSOU, Thierry, 88447 Birkenhard (DE); KONETZKI, Ingo, 88447 Warthausen (DE); LUSTENBERGER, Philipp, 88447 Warthausen (DE); MACK, Jürgen, 88400 Biberach (DE); SCHNAPP, Andreas, 88400 Biberach (DE); WIEDENMAYER, Dieter, 88400 Biberach (DE)
(74) Vertreter: Hammann, Heinz
(86) Internationale Anmeldenummer: PCT/EP2005/000245
(87) Internationale Veröffentlichungsnummer: WO 2005/070908

(56) Entgegenhaltungen:
- EP-A- 0 043 940
- WO-A-01/83462
- US-A- 4 215 119
- K. IAKOVOU ET. AL.: "Syntheis of oxypropanolamine derivatives of 3,4-dihydro-2H-1,4-benzoxazine, beta-adrenergic affinity, inotropic, chronotropic and coronary vasodilating activities." EUROPEAN JOURNAL OF MEDICINAL CHEMISTRY, Bd. 34, 1999, Seiten 903-917, XP002326858

## Beschreibung

Die vorliegende Erfindung betrifft Verbindungen der Formel **1** 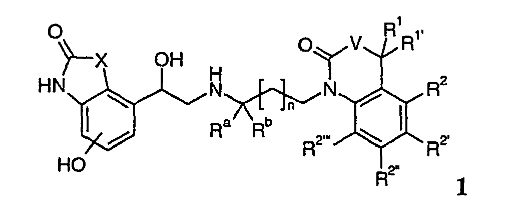 worin die Gruppen X, R^{a}, R^{b}, R¹, R^{1'}, R², R^{2'}, R^{2"}, R^{2"}, V und n die in den Ansprüchen und in der Beschreibung genannten Bedeutungen haben können, sowie deren Verwendung als Arzneimittel, insbesondere zur Behandlung von entzündlichen und obstruktiven Atemwegserkrankungen.

### Hintergrund der Erfindung

Betamimetika (β-adrenerge Substanzen) sind aus dem Stand der Technik-bekannt. Beispielsweise sei diesbezüglich auf die Offenbarung der EP 43 940 oder auch der WO 01/83462 verwiesen, die Betamimetika zur Therapie unterschiedlichster Erkrankungen vorschlagen.

Zur medikamentösen Therapie von Erkrankungen ist es häufig wünschenswert, Arzneimittel mit einer längeren Wirkungsdauer bereitzustellen. Hierdurch kann in der Regel gewährleistet werden, dass die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im Übrigen in hohem Maße zum Wohlbefinden des Patienten bei.

Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, dass der Patient sich relativ schnell an die regelmäßige Einnahme des Medikaments zu bestimmten Tageszeiten gewöhnen kann.

Es ist daher Aufgabe der vorliegenden Erfindung, Betamimetika bereitzustellen, die durch eine längere Wirkdauer gekennzeichnet sind und somit zur Herstellung von Arzneimitteln mit längerer Wirksamkeit Verwendung finden können. Es ist insbesondere Aufgabe der vorliegenden Erfindung, Betamimetika bereitzustellen, die aufgrund ihrer langen Wirksamkeit zur Herstellung eines einmal täglich applizierbaren Arzneimittels eingesetzt werden können. Ein weiteres Ziel der vorliegenden Erfindung ist die Bereitstellung von neuen Betamimetika, die aufgrund ihrer langen Wirksamkeit zur Herstellung eines einmal täglich applizierbaren Arzneimittels zur Behandlung von entzündlichen oder obstruktiven Atemwegserkrankungen verwendet werden können. Neben den vorstehend genannten Aufgaben ist es ferner Ziel der vorliegenden Erfindung, solche Betamimetika bereitzustellen, die nicht nur außerordentlich potent, sondern ferner durch ein hohes Maß an Selektivität gegenüber dem β2-Adrenozeptor gekennzeichnet sind.

### Detaillierte Beschreibung der Erfindung

Überraschenderweise wurde gefunden, dass die vorstehend genannten Aufgaben durch Verbindungen der Formel **1** gelöst werden. Dementsprechend betrifft die vorliegende Erfindung Verbindungen der Formel **1** 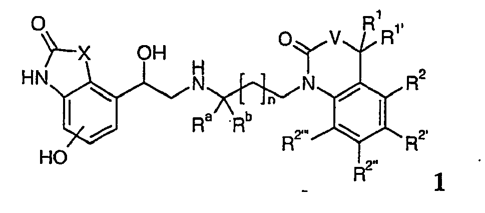 worin
- X: ein Gruppe -O-, -NH-, -CH₂-O-, -CHMe-O-, -C(Me)₂-O-, -CH₂-NH-, -CHMe-NH-, -C(Me)₂-NH-, -CH=CH- oder -CH₂-CH₂-;
- V: eine zweibindige Gruppe ausgewählt aus der Gruppe bestehend aus CH₂, NH und O, bevorzugt CH₂ und O, besonders bevorzugt O;
- R^{a} und R^{b}: gleich oder verschieden, ein Rest ausgewählt aus der Gruppe bestehend aus Wasserstoff, C₁₋₄-Alkyl, und Halogen-C₁₋₄-alkyl,
oder
- R^{a} und R^{b}: gemeinsam eine C₂₋₅-alkylen-Brücke, in der ein oder mehrere Wasserstoffatome gegebenenfalls ersetzt sein können durch Halogen;
- R¹ und R^{1'}: gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl, Halogen-C₃₋₆-cycloalkyl oder C₁₋₆-Alkylen-C₃₋₆-cycloalkyl, oder
- R¹ und R^{1'}: gemeinsam eine C₂₋₅-alkylen-Brücke in der ein oder mehrere Wasserstoffatome gegebenenfalls ersetzt sein können durch Halogen;
- R^{2'''}: gleich oder verschieden, ein Rest ausgewählt aus der Gruppe bestehend aus Wasserstoff, C₁₋₆-Alkyl, Halogen-C₁₋₆-alkylen, OH, HO-C₁₋₆-alkylen, -O-C₁₋₆-Alkyl, C₆₋₁₀-Aryl, C₆₋₁₀-Aryl-C₁₋₄-alkylen, C₆₋₁₀-Aryl-C₁₋₆-alkylen-O-, COOH, COOC₁₋₆-alkyl, O-C₁₋₆-alkylen-COOH, R², R^{2'}, R^{2''} und O-C₁₋₆-alkylen-COOC₁₋₆-alkyl, NHSO₂-C₁₋₆-alkyl, CN, NH₂, NH-C₁₋₆-Alkyl, N(C₁₋₆-Alkyl)₂,NO₂, S-C₁₋₆-Alkyl, SO₂-C₁₋₆-Alkyl, SO-C₁₋₆-Alkyl, O(CO)C₁₋₆-Alkyl, COC₁₋₆-Alkyl, NHCOC₁₋₆-Alkyl oder Halogen;
- n: 0, 1 oder 2; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Bevorzugt sind die Verbindungen der Formel **1**, worin
- X: -O-, -CH₂-O-, -C(Me)₂-O- oder -CH=CH-;
- V: eine zweibindige Gruppe ausgewählt aus der Gruppe bestehend aus CH₂, NH und O, bevorzugt CH₂ und O, besonders bevorzugt O;
- R^{a} und R^{b}: gleich oder verschieden, ein Rest ausgewählt aus der Gruppe bestehend aus Wasserstoff, C₁₋₄-Alkyl und Fluor-C₁₋₄-alkyl,
oder
- R^{a} und R^{b}: gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
- R¹ und R^{1'}: gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl oder C₁₋₆-Alkylen-C₃₋₆-Cycloalkyl,
oder
- R¹ und R^{1'}: gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
- R², R^{2'}, R^{2"} und R^{2"'}: gleich oder verschieden, ein Rest ausgewählt aus der Gruppe bestehend aus Wasserstoff, C₁₋₄-Alkyl, CF₃, CHF₂, CH₂F, OH, -O-C₁₋₄-Alkyl, Phenyl, Phenylethyl, Benzyl, Phenyloxy, Benzyloxy, COOH, COOC₁₋₄-alkyl, OCH₂COOH, OCH₂COOC₁₋₄-alkyl, NHSO₂-C₁₋₄-alkyl, Fluor, Chlor oder Brom;
- n: 0, 1 oder 2; bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind die Verbindungen der Formel 1, worin
- X: -O-, -CH₂-O-, -C(Me)₂-O- oder -CH=CH-;
- V: eine zweibindige Gruppe ausgewählt aus der Gruppe bestehend aus CH₂ und O, bevorzugt O;
- R^{a} und R^{b}: gleich oder verschieden, ein Rest ausgewählt aus der Gruppe bestehend aus Wasserstoff, Methyl, Ethyl und CF₃, bevorzugt Wasserstoff, Methyl oder Ethyl, oder
- R^{a} und R^{b}: gemeinsam eine Gruppe ausgewählt aus -CH₂-CH₂- und -CH₂-CH₂-CH₂-CH₂-, bevorzugt -CH₂-CH₂-;
- R¹ und R^{1'}: gleich oder verschieden, ein Rest ausgewählt aus der Gruppe bestehend aus Wasserstoff, Methyl, Ethyl, Propyl, Cyclopropyl oder Methylcyclopropyl, oder
- R¹ und R^{1'}: gemeinsam -CH₂-CH₂-CH₂-CH₂- oder -CH₂-CH₂-CH₂-CH₂-CH₂-;
- R², R^{2'}, R^{2"} und R^{2'"}: gleich oder verschieden, ein Rest ausgewählt aus der Gruppe bestehend aus Wasserstoff, Methyl, Ethyl, Propyl, CF₃, CHF₂, CH₂F, OH, Methyloxy, Ethyloxy, Propyloxy, COOH, COOCH₃, COOCH₂CH₃, OCH₂COOH, OCH₂COOCH₃, NHSO₂-CH₃, Fluor, Chlor oder Brom;
- n: 0, 1 oder 2; bevorzugt 1
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind die Verbindungen der Formel 1, in denen R^{a} und R^{b} beide Methyl und in denen die Gruppen X, R¹, R^{1'}, R², R^{2'}, R^{2"}, R^{2"'}, V und n die vorstehend genannten Bedeutungen haben können. Diese bevorzugten Verbindungen sind darstellbar durch die nachstehende allgemeine Formel **1.1** 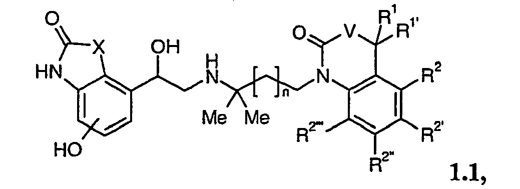 worin die Gruppen X, R¹, R^{1'}, R²,R^{2"} R^{2"}, R^{2"}, V und n die vorstehend genannten Bedeutungen haben können.

Von erfindungsgemäß besonderer Bedeutung sind die Verbindungen der Formel **1**, worin X der Gruppe -CH₂-O- entspricht. Diese Verbindungen sind darstellbar durch die Formel **1**' 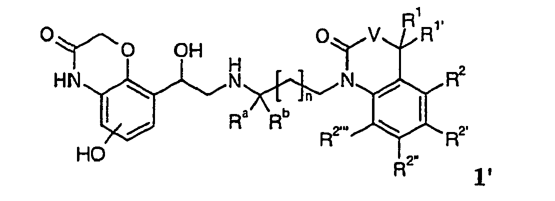 worin die Gruppen R^{a}, R^{b}, R¹, R^{1'}, R², R^{2'}, R^{2"}, R^{2"'} und V die oben stehende Bedeutung haben.

Bevorzugt sind Verbindungen der Formel **1**', worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl oder Cyclopropyl,
oder
- R¹ und R^{1'}: gemeinsam -CH₂-CH₂-CH₂-CH₂- oder -CH₂-CH₂-CH₂-CH₂-CH₂-;
- R² und R^{2'''}: Wasserstoff;
- R^{2'} und R^{2''}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Erfindungsgemäß bevorzugt sind Verbindungen der Formel 1', worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl, Propyl oder Cyclopropyl,
oder
- R¹ und R^{1'}: gemeinsam -CH₂-CH₂-CH₂-CH₂-CH₂-;
- R² und R^{2'"}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß gleichrangiger Bedeutung sind ferner die Verbindungen der Formel **1**', worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl, Ethyl oder Propyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß herausragender Bedeutung sind ferner die Verbindungen der Formel 1', worin R^{a} und R^{b} die vorstehend genannten Bedeutungen haben können und worin
- V: die zweibindige Gruppe O;
- R¹ und R^{1'}: jeweils gleichzeitig Wasserstoff, Methyl, Ethyl oder Propyl, sind;
- R², R^{2''} und R^{2'''}: Wasserstoff;
- R^{2'}: Wasserstoff, OH oder Methyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind feiner solche Verbindungen der Formel **1**', in denen R^{a} und R^{b} beide Methyl bedeuten. Diese Verbindungen sind darstellbar durch die Formel **1.1**' 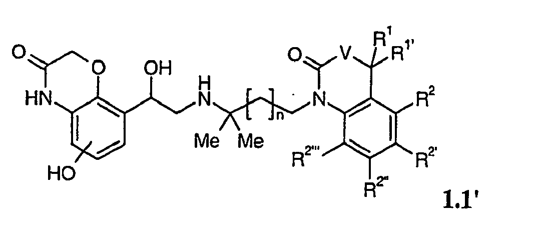 worin die Gruppen R¹, R^{1'}, R², R^{2'}, R^{2''}, R^{2'''} und V die vorstehend genannten Bedeutungen haben können.

Bevorzugt sind Verbindungen der Formel **1**, worin X der Gruppe -O- entspricht. Diese Verbindungen sind darstellbar durch die Formel **1**'' 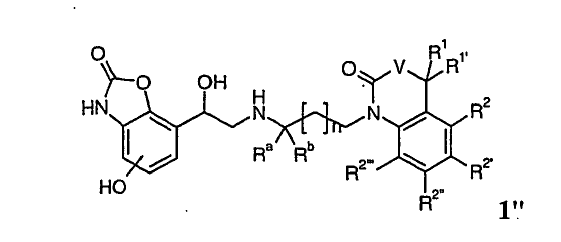 worin die Gruppen R^{a}, R^{b}, R¹, R^{1'}, R², R^{2'}, R^{2"}, R^{2'"} und V die oben stehende Bedeutung haben.

Besonders bevorzugt sind Verbindungen der Formel **1**", worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl, Cyclopropyl,
oder
- R¹ und R^{1'}: gemeinsam -CH₂-CH₂-CH₂-CH₂- oder -CH₂-CH₂-CH₂-CH₂-CH₂-;
- R² und R^{2'"}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind ferner Verbindungen der Formel **1**", worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl oder Propyl,
oder
- R¹ und R^{1'}: gemeinsam -CH₂-CH₂-CH₂-CH₂-CH₂-;
- R² und R^{2'''}: Wasserstoff;
- R^{2'} und R^{2''}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß gleichrangiger Bedeutung sind ferner die Verbindungen der Formel **1**", worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl oder Ethyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner solche Verbindungen der Formel **1**", in denen R^{a} und R^{b} beide Methyl bedeuten. Diese Verbindungen sind darstellbar durch die Formel 1.1" 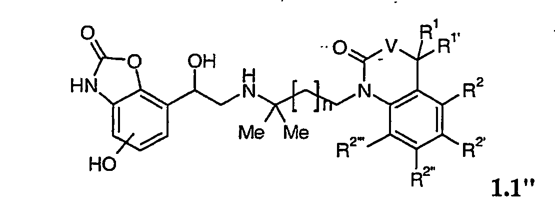 worin die Gruppen R¹, R^{1'}, R², R^{2'}, R^{2''}, R^{2'''} und V die vorstehend genannten Bedeutungen haben können.

Von erfindungsgemäß besonderer Bedeutung sind ferner Verbindungen der Formel **1**, worin X der Gruppe -CH=CH- entspricht. Diese Verbindungen sind darstellbar durch die Formel **1**"' 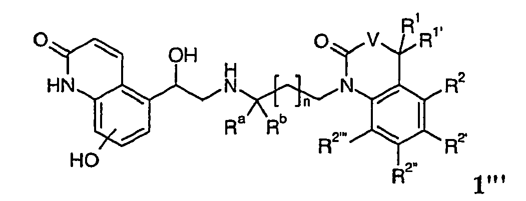 worin die Gruppen R^{a}, R^{b}, R¹, R^{1'}, R², R^{2'}, R^{2''}, R^{2'''} und V die oben stehende Bedeutung haben.

Bevorzugt sind Verbindungen der Formel **1**"', worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl, Cyclopropyl, bevorzugt Methyl oder Ethyl,
oder
- R¹ und R^{1'}: gemeinsam -CH₂-CH₂-, -CH₂-CH₂-CH₂-CH₂- oder -CH₂-CH₂-CH₂-CH₂-CH₂-;
- R²und R^{2'"}: Wasserstoff;
- R^{2'} und R^{2"}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind Verbindungen der Formel **1**"', worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R¹': gleich oder verschieden Wasserstoff Methyl, Ethyl oder Propyl,
oder
- R¹ und R^{1'}: gemeinsam -CH₂-CH₂-, -CH₂-CH₂-CH₂-CH₂- oder -CH₂-CH₂-CH₂-CH₂-CH₂-;
- R² und R^{2'"}: Wasserstoff;
- R^{2'}und R^{2"}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind ferner Verbindungen der Formel **1**"', worin R^{a} und R^{b} die vorstehend genannten Bedeutungen haben können und worin
- V: die zweibindige Gruppe O;
- R¹ und R¹': gleich oder verschieden, bevorzugt gleich Wasserstoff, Methyl, Ethyl oder Propyl;
- R², R^{2''} und R^{2''}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß herausragender Bedeutung sind ferner die Verbindungen der Formel 1"', worin R^{a} und R^{b} die vorstehend genannten Bedeutungen haben können und worin
- V: die zweibindige Gruppe O;
- R¹ und R^{1'}: jeweils gleichzeitig Wasserstoff, Methyl, Ethyl oder Propyl sind;
- R², R^{2"} und R^{2'''}: Wasserstoff;
- R^{2'}: Wasserstoff, OH oder Methyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner solche Verbindungen der Formel **1**''', in denen R^{a} und R^{b} beide Methyl bedeuten. Diese Verbindungen sind darstellbar durch die Formel 1.1 "' 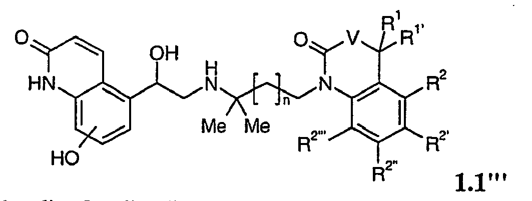 worin die Gruppen R¹, R^{1'}, R², R^{2'}, R^{2"}, R^{2'''} und V die vorstehend genannten Bedeutungen haben können.

Von erfindungsgemäß besonderer Bedeutung sind ferner Verbindungen der Formel **1**, worin X für die Gruppe -CMe₂-O- steht. Diese Verbindungen sind darstellbar durch die Formel **1""** 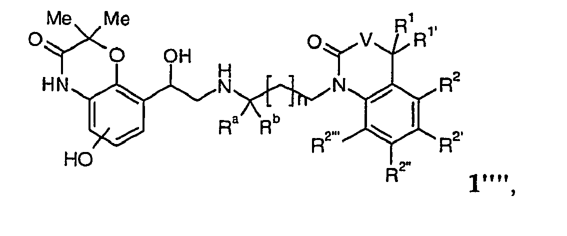 worin die Gruppen R^{a}, R^{b}, R¹, R^{1'}, R², R^{2'}, R^{2"}, R^{2'''} und V die oben stehende Bedeutung haben.

Bevorzugt sind Verbindungen der Formel **1**"", worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden, ein Rest ausgewählt aus der Gruppe bestehend aus Wasserstoff, Methyl, Ethyl, Propyl, Cyclopropyl,
oder
- R¹ und R^{1'}: gemeinsam -CH₂-CH₂-CH₂-CH₂- oder -CH₂-CH₂-CH₂-CH₂-CH₂-;
- R² und R^{2'''}: Wasserstoff;
- R^{2'} und R^{2''}: gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind Verbindungen der Formel 1"", worin R^{a} und R^{b} die vorstehend genannten Bedeutungen haben können und worin
- V: eine zweibindige Gruppe ausgewählt aus der Gruppe bestehend aus CH₂ und O, bevorzugt O;
- R¹ und R^{1'}: gleich oder verschieden Wasserstoff Methyl, Ethyl oder Propyl
oder
- R¹ und R^{1'}: gemeinsam -CH₂-CH₂-CH₂-CH₂-CH₂-;
- R² und R^{2'''}: Wasserstoff;
- R^{2'} und R^{2''}: gleich oder verschieden, ein Rest ausgewählt aus der Gruppe bestehend aus Wasserstoff, Methyl, CF₃, OH, Methyloxy, Benzyloxy oder Fluor;
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugt sind ferner die Verbindungen der Formel **1""**, worin R^{a} und R^{b} die vorstehend genannten Bedeutungen haben können und worin
- V: die zweibindige Gruppe O;
- R¹ und R^{1'}: gleich oder verschieden, bevorzugt gleich Wasserstoff, Methyl, Ethyl oder Propyl;
- R², R^{2"} und R^{2"}: Wasserstoff;
- R^{2'}: Wasserstoff, OH, Methyloxy oder Benzyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß herausragender Bedeutung sind ferner die Verbindungen der Formel **1"",** worin R^{a} und R^{b} die vorstehend genannten Bedeutungen haben können und worin
- V: die zweibindige Gruppe O ;
- R¹ und R^{1'}: jeweils gleichzeitig Wasserstoff, Methyl, Ethyl oder Propyl sind;
- R², R^{2"} und R^{2'"}: Wasserstoff;
- R^{2'}: Wasserstoff, OH oder Methyloxy,
- n: 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Von erfindungsgemäß besonderer Bedeutung sind ferner solche Verbindungen der Formel **1**"", in denen R^{a} und R^{b} beide Methyl bedeuten. Diese Verbindungen sind darstellbar durch die Formel 1.1"" 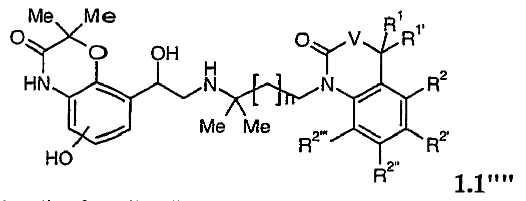 worin die Gruppen R¹, R^{1'}, R², R^{2'}, R^{2"}, R^{2'"} und V die vorstehend genannten Bedeutungen haben können.

Von erfindungsgemäß herausragender Bedeutung sind ferner die Verbindungen der Formel 1, worin
- R¹ und R^{1'}: jeweils gleichzeitig Wasserstoff, Methyl oder Ethyl, bevorzugt Methyl oder Ethyl, besonders bevorzugt Ethyl sind:
und die Gruppen X, R², R^{2'}, R^{2"} , R^{2"'}, V und n eine der vorstehend genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Ebenfalls von erfindungsgemäß herausragender Bedeutung sind ferner die Verbindungen der Formel **1**, worin
- n: 1
und die Gruppen X, R¹, R^{1'}, R², R^{2'}, R^{2"}, R^{2"'} und V eine der vorstehend genannten Bedeutungen haben können, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

Besonders bevorzugte Verbindungen der allgemeinen Formel 1 sind ausgewählt aus der Gruppe bestehend aus:
- 1-{3-[2-Hydroxy-2-(6-hydroxy-3-oxo-3,4-dihydro-2H-benzo[1,4]oxazin-8-yl)-ethylamino]-3-methyl-butyl }-6-methoxy-4,4-dimethyl-1,4-dihydro-benzo[d] [1 ,3]oxazin-2-on,
- 6-Hydroxy-1-{3-[2-hydroxy-2-(6-hydroxy-3-oxo-3,4-dihydro-2H-benzo[1,4]oxazin-8-yl)-ethylamino]-3-methyl-butyl}-4,4-dimethyl-1,4-dihydro-benzo[d][1,3]oxazin-2-on,
- 4,4-Diethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[d][1,3]oxazin-2-on,
- 6-Hydroxy-8-{1-hydroxy-2-[3-(3-hydroxy-4,4-dimethyl-1-oxo-3,4-dihydro-1H-isoquinolin-2-yl)-1,1-dimethyl-propylamino]-ethyl }-4H-benzo[1,4]oxazin-3-on,
- 1-{3-[2-Hydroxy-2-(6-hydroxy-3-oxo-3,4-dihydro-2H-benzo[1,4]oxazin-8-yl)-ethylamino]-3-methyl-butyl -1,4-dihydro-benzo[1,3]oxazin-2-on,
- 4-Ethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on,
- 8-(2-[1,1-Dimethyl-3-(2-oxo-3,4-dihydro-2H-chinolin-1-yl)-propylamino]-1-hydroxyethyl}-6-hydroxy-4H-benzo[1,4]oxazin-3-on,
- 4,4-Diethyl-1- {3-[2-hydroxy-2-(8-hydroxy-2-oxo-1,2-dihydro-chinolin-5-yl)-ethylamino]-3-methyl-butyl -1,4-dihydro-benzo[1,3]oxazin-2-on,
- 4,4-Diethyl-1-{3-[2-hydroxy-2-(6-hydroxy-2,2-dimethyl-3-oxo-3,4-dihydro-2H-benzo[1,4]oxazin-8-yl)-ethylamino]-3-methyl-butyl}-1,4-dihydro-benzo[d][1,3]oxazin-2-on,
- 4,4-Diethyl-1-{3-[2-hydroxy-2-(6-hydroxy-2,2-dimethyl-3-oxo-3,4-dihydro-2H-benzo[1,4]oxazin-8-yl)-ethylamino]-3-methyl-butyl}-1,4-dihydro-benzo[d][1,3]oxazin-2-on,
- 4,4-Diethyl-1-{3-[2-hydroxy-2-(7-hydroxy-2-oxo-1,2-dihydro-quinolin-5-yl)-ethylamino]-3-methyl-butyl}-1,4-dihydro-benzo[d][1,3]oxazin-2-on,
- 1-{3-[2-Hydroxy-2-(6-hydroxy-3-oxo-3,4-dihydro-2*H*-benzo[1,4]oxazin-8-yl)-ethylamino]-3-methyl-butyl }-4,4-dipropyl-1,4-dihydro-benzo[d][1,3]oxazin-2-on,
- 1-{3-[2-Hydroxy-2-(5-hydroxy-3-oxo-3,4-dihydro-2*H*-benzo[1,4]oxazin-8-yl)-ethylamino]-3-methyl-butyl }-4,4-dipropyl-1,4-dihydro-benzo[*d*] [1,3]oxazin-2-on,
- 4,4-Diethyl-7-fluor-1-{3-[2-hydroxy-2-(6-hydroxy-3-oxo-3,4-dihydro-2*H-*benzo[1,4]oxazin-8-yl)-ethylamino]-3-methyl-butyl}-1,4-dihydro-benzo[*d*][1,3]oxazin-2-on,
- 4,4-Diethyl-1-{3-[2-hydroxy-2-(5-hydroxy-3-oxo-3,4-dihydro-2*H*-benzo[1,4]oxazin-8-yl)-ethylamino] -3-methyl-butyl}-8 -methoxy-1,4-dihydro-benzo[*d*] [1,3]oxazin-2-on,
- 1-(2-{1-[2-Hydroxy-2-(5-hydroxy-3-oxo-3,4-dihydro-2H-benzo[1,4]oxazin-8-yl)-ethylamino]-cyclopropyl }-ethyl)-4,4-dimethyl-1,4-dihydro-benzo[d] [1,3]oxazin-2-on,
- 1-(2-{ 1-[2-Hydroxy-2-(6-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on, .
- 1-{3-[2-Hydroxy-2-(6-hydroxy-3-oxo-3,4-dihydro-2*H*-benzo[1,4]oxazin-8-yl)-ethylamino]-3-methyl-butyl}-spiro(cyclohexan-1,4'-2H-3',1'-benzoxazin)-2'-on,
- 1-{3-[2-Hydroxy-2-(5-hydroxy-3-oxo-3,4-dihydro-2*H*-benzo[1,4]oxazin-8-yl)-ethylamino]-3-methyl-butyl}-spiro(cyclohexan-1,4'-2H-3',1'-benzoxazin)-2'-on;
- 4,4-Dimethyl-1-{ 3-[2-hydroxy-2-(6-hydroxy-3-oxo-3,4-dihydro-2H-benzo[1,4]oxazin-8-yl)-ethylamino]-propyl}-1,4-dihydro-benzo[d][1,3]oxazin-2-on;
- 4,4-Dimethyl-1-{ 3-[2-hydroxy-2-(5-hydroxy-3-oxo-3,4-dihydro-2H-benzo[1,4]oxazin-8-yl)-ethylamino]-propyl}-1,4-dihydro-benzo[d][1,3]oxazin-2-on,
gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren

Die OH-Gruppe kann in den vorstehend definierten Verbindungen der Formel 1 in drei unterschiedlichen Positionen konfiguriert sein. Die bevorzugten Isomere sind durch die nachstehenden allgemeinen Formeln 1a und 1b darstellbar, 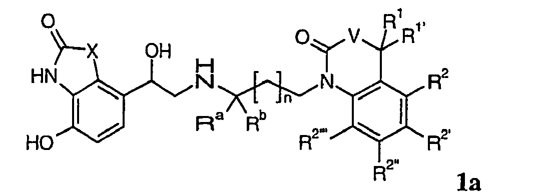 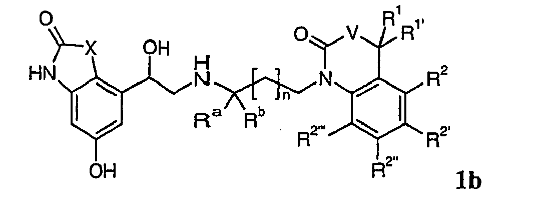 worin die Gruppen X, R^{a}, R^{b}, R¹, R^{1'}, R², R^{2'}, R^{2"}, R^{2"'}, V und n die vorstehend genannten Bedeutungen haben können.

Besonders bevorzugte Verbindungen sind insbesondere auch solche der allgemeinen Formel **1.1'-b** 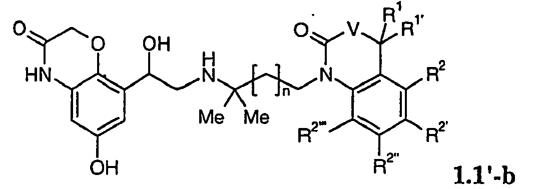 worin die Gruppen R¹, R^{1'}, R², R^{2'}, R^{2"}, R^{2'''} und V die vorstehend genannten Bedeutungen haben können.

Besonders bevorzugte Verbindungen sind insbesondere auch solche der allgemeinen Formel **1.1"-b** 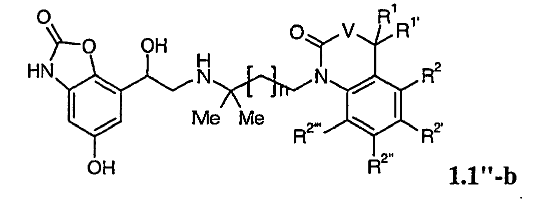 worin die Gruppen R¹, R^{1'}, R², R^{2'}, R^{2"}, R^{2'"} und V die vorstehend genannten Bedeutungen haben können.

Besonders bevorzugte Verbindungen sind insbesondere auch solche der allgemeinen Formel **1.1"'-a** 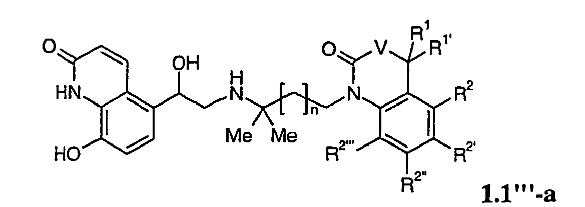 worin die Gruppen R¹, R^{1'}, R², R^{2'}, R^{2"}, R^{2"'} und V die vorstehend genannten Bedeutungen haben können.

Besonders bevorzugte Verbindungen sind insbesondere auch solche der allgemeinen Formel **1.1""-b** 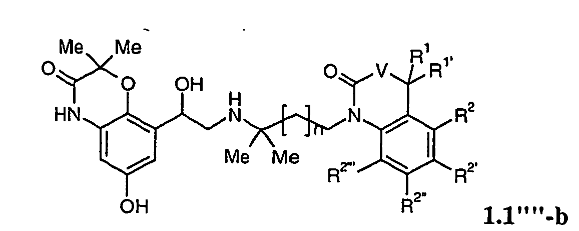 worin die Gruppen R¹, R^{1'}, R², R^{2'} , R^{2"}, R^{2"'} und V die vorstehend genannten Bedeutungen haben können.

Die Verbindungen der Formel **1** können gegebenenfalls in Form der einzelnen optischen Isomeren, Mischungen der einzelnen Enantiomeren oder Racemate zur Anwendung gelangen. Besonders bevorzugt gelangen Sie in Form der enantiomerenreinen Verbindungen zur Anwendung, wobei die Verbindungen der Formel **1**, in denen das zum Phenylring benzylische, asymmetrische Kohlenstoffzentrum "-CH(OH)-" R-konfiguriert ist. Die besonders bevorzugten R-Enantiomere der Verbindungen der allgemeinen Formel **1** sind durch die allgemeine Formel ***R*-1** darstellbar, 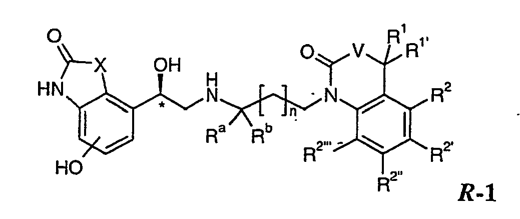 worin die Gruppen X, R^{a}, R^{b}, R¹, R^{1'}, R², R^{2'}, R^{2"}, R^{2"'}, V und n die vorstehend genannten Bedeutungen haben können.

Unter Säureadditionssalzen mit pharmakologisch unbedenklichen Säuren werden dabei beispielsweise Salze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrobenzoat, Hydrocitrat, Hydrofumarat, Hydrotartrat, Hydrooxalat, Hydrosuccinat, Hydrobenzoat und Hydro-*p*-toluolsulfonat, bevorzugt Hydrochlorid, Hydrobromid, Hydrosulfat, Hydrophosphat, Hydrofumarat und Hydromethansulfonat verstanden.

Halogen steht im Rahmen der vorliegenden Erfindung für Fluor, Chlor, Brom oder Jod. Sofern nicht gegenteilig angegeben, gelten Fluor und Brom als bevorzugte Halogene, wobei Fluor im Allgemeinen bevorzugt ist.

Als Alkylgruppen (Alkyl) werden, soweit nicht anders angegeben, verzweigte und unverzweigte Alkylgruppen mit 1 bis 6, bevorzugt 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, sec-Butyl und tert-Butyl etc.

Als Alkylengruppen (Alkylen) werden, soweit nicht anders angegeben, verzweigte und unverzweigte Alkylengruppen mit 1 bis 6, bevorzugt 1 bis 4 Kohlenstoffatomen bezeichnet. Beispielsweise werden genannt: Methylen, Ethylen, Propylen oder Butylen. Sofern nicht anders beschrieben, umfassen die Definitionen Propylen und Butylen alle denkbaren isomeren Formen der jeweiligen Reste.

Als Cycloalkylgruppen (Cycloalkyl) werden, soweit nicht anders angegeben cyclische Alkylgruppen mit 3 bis 6 bezeichnet. Beispielsweise werden genannt: Cyclopropyl, Cyclobutanyl, Cyclopentyl oder Cyclohexyl.

Als Alkyloxygruppen (O-Alkyl) werden, soweit nicht anders angegeben, verzweigte und unverzweigte Alkylgruppen mit 1 bis 6, bevorzugt 1 bis 4 Kohlenstoffatomen bezeichnet, die über ein Sauerstoffatom verknüpft sind. Beispielsweise werden genannt: Methyloxy, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen -OMe, -OEt, -OProp oder -OBu verwendet. Sofern nicht anders beschrieben, umfassen die Definitionen Propyloxy und Butyloxy alle denkbaren isomeren Formen der jeweiligen Reste. So umfasst beispielsweise Propyloxy *n*-Propyloxy und *iso*-Propyloxy, Butyloxy umfasst *iso*-Butyloxy, *sec*-Butyloxy und *tert*-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Halogenalkylengruppen werden, soweit nicht anders angegeben, verzweigte und unverzweigte Alkylgruppen mit 1 bis 6 Kohlenstoffatomen bezeichnet, bei denen ein oder mehrere Wasserstoffatome durch Halogenatome, bevorzugt durch Fluor ersetzt sind. Beispielsweise werden genannt: CHF₂, CF₃, CH₂CF₃, CF₂CF₃.

Als Arylgruppen werden, soweit nicht anders angegeben, aromatische Ringsysteme mit 6 bis 10 Kohlenstoffatomen bezeichnet. Bevorzugte Arylgruppen sind Phenyl und Naphthyl, wobei Phenyl erfindungsgemäß besonders bevorzugt ist.

Als Arylalkylengruppen werden, soweit nicht anders angegeben, vorstehend genannte Arylgruppen bezeichnet, die über verzweigte und unverzweigte Alkylgruppen mit 1 bis 4 Kohlenstoffatomen verknüpft sind. Beispielsweise werden genannt Benzyl, Phenylethyl, Naphthylmethyl, Naphthylethyl. Die verbrückenden Alkylgruppen werden im Rahmen der vorliegenden Erfindung auch als Alkylenbrücken bezeichnet.

Als Aryloxygruppen (O-Aryl) werden, soweit nicht anders angegeben, Arylgruppen mit 6 bis 10 Kohlenstoffatomen bezeichnet, die über eine Sauerstoffbrücke verknüpft sind. Bevorzugt Gruppen sind in diesem Zusammenhang beispielsweise Phenyloxy oder Naphthyloxy, die im Rahmen der vorliegenden Erfindung gegebenenfalls auch als Phenoxy oder Naphthoxy bezeichnet werden können.

Als Arylalkylenoxygruppen (Arylalkylen-O-) werden, soweit nicht anders angegeben, Arylgruppen bezeichnet, die über verzweigte und unverzweigte Alkyloxygruppen mit 1 bis 4 Kohlenstoffatomen verknüpft sind. Beispielsweise werden genannt Benzyloxy, Phenylethyloxy, Naphthylmethyloxy, Naphthylethyloxy.

Die Herstellung der erfindungsgemäßen Verbindungen kann in Analogie zu im Stand der Technik bereits bekannten Vorgehensweisen erfolgen. Geeignete Herstellverfahren sind beispielsweise aus der EP 43 940 oder auch aus der WO O1/83462 bekannt, auf die an dieser Stelle vollinhaltlich Bezug genommen wird.

Die nachstehend beschriebenen Synthesebeispiele dienen der weitergehenden Illustration von neuen erfindungsgemäßen Verbindungen. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

### Beispiel 1: 1-{3-[2-Hydroxy-2-(6-hydroxy-3-oxo-3,4-dihydro-2H-benzo[1,4]oxazin-8-yl)-ethylamino]-3-methyl-butyl} -6-methoxy-4,4-dimethyl-1,4-dihydro-benzo[d][1,3]oxazin-2-on

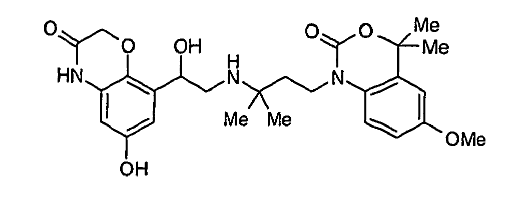

### a) (2-Acetyl-4-methoxy-phenyl)-carbaminsäureethylester

Zu einer Lösung von 82.5 g (0.5 mol) 2-Amino-5-methoxyacetophenon in 400 mL Pyridin werden 65.1 g (0.6 mol) Chlorameisensäureethylester unter Kühlung getropft, so dass die Temperatur nicht über 10-15°C steigt. Anschließend wird die Reaktionsmischung 2 h bei Raumtemperatur gerührt und dann auf Eis gegossen. Der ausfallende Niederschlag wird abgesaugt, mit Wasser gewaschen und aus Isopropanol umkristallisiert. Ausbeute: 102 g (86%); Smp. = 97-100°C.

### b) 6-Methoxy-4,4-dimethyl-1,4-dihydro-benzo[1,3]oxazin-2-on

Zu einer Lösung von 0.5 mol Methylmagnesiumiodid in 200 mL Diethylether werden 47.4 g (0.2 mol) (2-Acetyl-4-methoxy-phenyl)-carbaminsäureethylester, gelöst in 275 mL THF, unter Kühlung so zugetropft, dass die Temperatur nicht über 0°C steigt. Man lässt 30 Minuten bei Raumtemperatur und anschließend 2 Stunde unter Rückfluss rühren. Die Reaktionsmischung wird auf Eis gegossen und mit Ammoniumchlorid versetzt. Nach dem Abtrennen der organischen Phase wird wiederholt mit Ethylacetat extrahiert. Die organischen Phasen werden vereinigt, mit Wasser gewaschen, mit Natriumsulfat getrocknet und eingeengt. Der Rückstand wird in Methanol gelöst und die Lösung wird eingeengt und dann mit Wasser versetzt. Der dabei ausfallende Niederschlag wird abgetrennt, mit Wasser gewaschen und aus Toluol umkristallisiert. Ausbeute: 31.1 g (75%); Smp. = 178-180°C.

### c) 1-(3-Amino-3-methyl-butyl)-6-methoxy-4,4-dimethyl-1,4-dihydro-benzo[1,3]oxazin-2-on

Eine Lösung von 31 g (0.15 mol) 6-Methoxy-4,4-dimethyl-1,4-dihydro-benzo[1,3]oxazin-2-on in 120 mL HMPT wird bei 65-70°C zu 7.2 g Natriumhydrid (55-60 %ig) in 30 mL HMPT getropft. Nach beendeter Wasserstofffreisetzung wird noch 20 Minuten nachgerührt und dann auf Raumtemperatur abgekühlt. Bei dieser Temperatur werden 37.7 g (0.18 mol) (3-Chlor-1,1-dimethyl-propyl)-benzylidenamin, gelöst in 40 mL HMPT, zugegeben. Nach 3 Stunden Rühren bei 100°C wird die Reaktionsmischung auf Eis gegossen und mit Ethylacetat extrahiert. Die organischen Phasen werden mit Wasser gewaschen, mit Natrumsulfat getrocknet und eingeengt. Der Rückstand wird unter Erwärmung in 1 N Salzsäure gelöst und nach Abkühlung mit Diethylether extrahiert. Die wässrige Phase wird mit Natronlauge alkalisch gestellt und mit Ethylacetat ausgeschüttelt. Anschließend wird die organische Phase mit Natriumsulfat getrocknet und vom Lösungsmittel befreit. Aus dem Rückstand wird nach Lösen in Acetonitril und Zugabe von etherischer Salzsäure das Produkt in Form seines Hydrochlorids isoliert. Ausbeute: 34.3 g (70 %).

### d) 1-{3-[2-(6-Benzyloxy-3-oxo-3,4-dihydro-2H-benzo[1,4]oxazin-8-yl)-2-hydroxy-ethylamino]-3-methyl-butyl}-6-methoxy-4,4-dimethyl-1,4-dihydro-benzo[1,3]oxazin-2-on

357 mg (1 mmol) 6-Benzyloxy-8-(2-ethoxy-2-hydroxy-acetyl)-4H-benzo[1,4]oxazin-3-on und 292 mg (1 mmol) 1-(3-Amino-3-methyl-butyl)-6-methoxy-4,4-dimethyl-1,4-dihydro-benzo[1,3]oxazin-2-on werden in 5 mL Ethanol suspendiert und auf 70°C erwärmt. Die entstandene Lösung wird eine Stunde bei 70°C gerührt und dann auf Raumtemperatur abgekühlt. Nach Zugabe von 113 mg (3 mmol) Natriumborhydrid wird 3 Stunden bei Raumtemperatur gerührt, mit 0.7 mL gesättigter Kaliumcarbonatlösung versetzt und weitere 30 Minuten gerührt. Es wird über Aluminiumoxid (basisch) filtriert, wiederholt mit Methylenchlorid/Methanol 15:1 nachgewaschen und eingeengt. Das so erhaltene Rohprodukt wird chromatographisch (Methylenchlorid mit Methanol/Ammoniak-Gradient (9:1)) gereinigt. Beigefarbener Feststoff. Ausbeute: 340 mg (58%); Massenspektrometrie: [M+H]⁺ = 590.

### e) 1-{3-(2-Hydroxy-2-(6-hydroxy-3-oxo-3,4-dihydro-2H-benzo[1,4]oxazin-8-yl)-ethylamino]-3-methyl-butyl }-6-methoxy-4,4-dimethyl-1,4-dihydro-benzo[d] [1,3]oxazin-2-on

340 mg (0.58 mmol) 1-{3-[2-(6-Benzyloxy-3-oxo-3,4-dihydro-2H-benzo[1,4]oxazin-8-yl)-2-hydroxy-ethylamino]-3-methyl-butyl }-6-methoxy-4,4-dimethyl-1,4-dihydro-benzo[1,3]oxazin-2-on werden in 10 mL Methanol gelöst und mit Palladium auf Kohle als Katalysator bei 1 bar Wasserstoffdruck hydriert. Anschließend wird der Katalysator abfiltriert und das Filtrat eingeengt. Beigefarbener Feststoff. Ausbeute: 273 mg (95%); Massenspektrometrie: [M+H]⁺ = 500; Rf-Wert = 0.33 (Methylenchlorid : Methanol : Ammoniak = 9 : 1 : 0.1).

### Beispiel 2: 6-Hydroxy-1-{3-[2-hydroxy-2-(6-hydroxy-3-oxo-3,4-dihydro-2H-benzo[1.4]oxazin-8-yl)-ethylamino]-3-methyl-butyl}-4,4-dimethyl-1,4-dihydro-benzo[d][1,3]oxazin-2-on

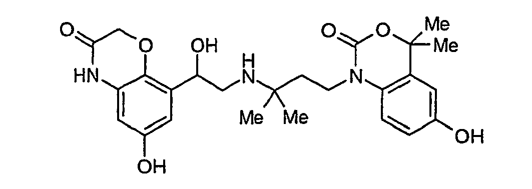

### a) 6-Benzyloxy-4,4-dimethyl-1,4-dihydro-benzo[1,3]oxazin-2-on

Die Herstellung erfolgt in Analogie zur Vorschrift für Beispiel 1b) aus 15.7 g (50 mmol) (2-Acetyl-4-benzyloxy-phenyl)-carbaminsäureethylester und 125 mmol Methylmagnesiumiodid. Ausbeute: 10.8 g (76%); Smp. = 134°C.

### b) 1-(3-Amino-3-methyl-bulyl)-6-benzyloxy-4,4-dimethyl-1,4-dihydro-benzo[1,3]oxazin-2-on

In Analogie zur Vorschrift für Beispiel 1c) aus 10.5 g (37 mmol) 6-Benzyloxy-4,4-dimethyl-1,4-dihydro-benzo[1,3]oxazin-2-on und 9:3 g (44 mmol) (3-Chlor-1,1-dimethylpropyl)-benzylidenamin hergestellt. Ausbeute: 10.9 g (73%); Smp. = 233°C (Hydrochlorid).

### c) 6-Hydroxy-1-{3-(2-hydroxy-2-(6-hydroxy-3-oxo-3,4-dihydro-2H-benzo[1,4]oxazin-8-yl)-ethylamino]-3-methyl-butyl }-4,4-dimethyl-1,4-dihydro-benzo[1,3]oxazin-2-one

Die Umsetzung von 357 mg (1 mmol) 6-Benzyloxy-8-(2-ethoxy-2-hydroxy-acetyl)-4H-benzo[1,4]oxazin-3-on und 368 mg (1 mmol) 1-(3-Amino-3-methyl-butyl)-6-benzyloxy-4,4-dimethyl-1,4-dihydro-benzo[1,3]oxazin-2-on in Analogie zu den Vorschriften für Beispiel 1 liefert die Verbindung in Form eines beigefarbenen Feststoffes. Ausbeute 355 mg (73%); Massenspektrometrie: [M+M]⁺ = 486.

### Beispiel 3: 4,4-Diethyl-1-{3-[2-hydroxy-2-(6-hydroxy-3-oxo-3.4-dihydro-2H-benzo[1,4]oxazin-8-yl)-ethylamino]-3-methyl-butyl }-1 4-dihydro-benzo[d][1,]oxazin-2-on

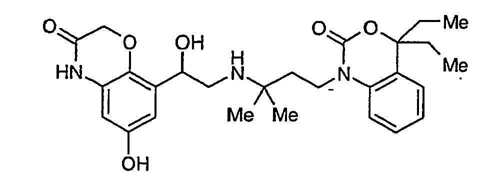

### a) 4,4-Diethyl-1,4-dihydro-benzo[1,3]oxazin-2-on

Erhalten aus der Reaktion von 67 g (0.3 mol) 2-Ethoxycarbonylamino-benzoesäuremethylester und 1.14 mol Ethylmagnesiumiodid in Analogie zur Vorschrift für Beispiel 1b). Ausbeute: 48.5 g (79%); Smp. = 160-162°C.

### b) 1-(3-Amino-3-methyl-butyl)-4,4-diethyl-1,4-dihydro-benzo[1,3]oxazin-2-on

Hergestellt aus 47.5 g (0.23 mol) 4,4-Diethyl-1,4-dihydro-benzo[1,3]oxazin-2-on und 57.5 g (0.27 mol) (3-Chlor-1,1-dimethyl-propyl)-benzylidenamin nach dem für Beispiel 1c) beschriebenen Verfahren. Ausbeute: 38.1 g (50%); Smp. = 208-210°C (Hydrochlorid).

### c) 4,4-Diethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[d][1,3]oxazin-2-on

357 mg (1 mmol) 6-Benzyloxy-8-(2-ethoxy-2-hydroxy-acetyl)-4H-benzo[1,4]oxazin-3-on und 290 mg (1 mmol) 1-(3-Amino-3-methyl-butyl)-4,4-diethyl-1,4-dihydro-benzo[1,3]oxazin-2-on werden in Analogie zu den Vorschriften für Beispiel 1 umgesetzt.

Nach anschließender Debenzylierung wird ein beigefarbener Feststoff erhalten. Ausbeute: 367 mg (74%); Massenspektrometrie: [M+H]⁺ = 498.

### Beispiel 4: 1-{3-[2-Hydroxy-2-(6-hydroxy-3-oxo-3,4-dihydro-2H-benzo[1,4]oxazin-8-yl)-ethylamino]-3-methyl-butyl}-4,4-dimethyl-1,4-dihydro-benzo[d] [1,3]oxazin-2-on

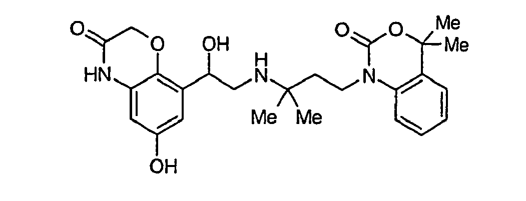

### a) 4,4-Dimethyl-1,4-dihydro-benzo[1,3]oxazin-2-on

112 g (1.13 mol) Phosgen werden in 500 mL THF eingeleitet. Anschließend wird eine Lösung von 52 g (0.34 mol) 2-(2-Amino-phenyl)-propan-2-ol, hergestellt aus 2-Aminoacetophenon und Methylmagnesiumiodid, in 300 mL THF zugegeben. Die Reaktionsmischung wird über Nacht stehen gelassen, eingeengt und mit 500 ml Pyridin versetzt. Nach dem Abdestillieren des Pyridins wird mit Wasser versetzt und mit Diethylether extrahiert. Die organischen Phasen werden nacheinander mit 2 N Salzsäure, Natriumhydroxid-Lösung und Wasser gewaschen, mit Natriumsulfat getrocknet und eingeengt. Der verbleibende Rückstand (46 g) wird ohne weitere Aufreinigung direkt weiter umgesetzt. Smp. (Toluol/Petrolether) = 109-110°C.

### b) 1-(3-Amino-3-methyl-butyl)-4,4-dimethyl-1,4-dihydro-benzo[1,3]oxazin-2-on

Erhalten aus 43 g (0.24 mol) 4,4-Dimethyl-1,4-dihydro-benzo[1,3]oxazin-2-on und 54 g (0.26 mol) (3-Chlor-1,1-dimethyl-propyl)-benzylidenamin in Analogie zu der für Beispiel 1c) beschriebenen Verfahren. Ausbeute 41 g (57%); Smp. (nach Umkristallisation aus Ethanol) = 262°C (Hydrochlorid).

### c) 1-{3-[2-Hydroxy-2-(6-hydroxy-3-oxo-3,4-dihydro-2H-benzo[1,4]oxazin-8-yl)-ethylamino]-3-methyl-butyl}-4,4-dimethyl-1,4-dihydro-benzo[d][1,3]oxazin-2-on

357 mg (1 mmol) 6-Benzyloxy-8-(2-ethoxy-2-hydroxy-acetyl)-4H-benzo[1,4]oxazin-3-on und 262 mg (1 mmol) 1-(3-Amino-3-methyl-butyl)-4,4-dimethyl-1,4-dihydro-benzo[1,3]oxazin-2-on werden in der für Beispiel 1 ausgeführten Weise umgesetzt. Nach anschließender Hydrierung wird ein beigefarbener Feststoff isoliert. Ausbeute: 285 mg (61%); Massenspektrometrie [M+H]⁺ = 470.

### Beispiel 5: 1-{3-[2-Hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on

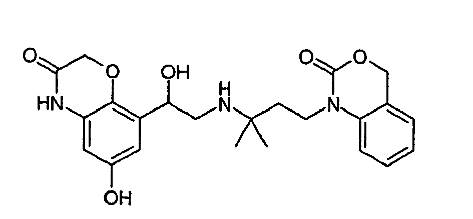

### a)1-(3-Amino-3-methyl-butyl)-1,4-dihydro-benzor[1,3]oxazin-2-on

2.70 g (18 mmol) 1,4-Dihydro-benzo[1,3]oxazin-2-on und 4.35 g (21 mmol) (3-Chlor-1,1-dimethyl-propyl)-(1-phenyl-methyliden)-amin werden in der für Beispiel 6a) beschriebenen Weise umgesetzt. Zur Aufarbeitung wird das Reaktionsgemisch auf Eiswasser gegossen und mit Ethylacetat extrahiert. Die organischen Phasen werden mit Wasser gewaschen, mit Natriumsulfat getrocknet und eingeengt. Der Rückstand wird mit 25 mL 2 N Salzsäure versetzt und auf 70°C erwärmt. Nach Abkühlung auf Raumtemperatur wird mit Diethylether extrahiert. Die wässrige Phase wird eingeengt und mit Acetonitril versetzt. Der ausfallende Niederschlag wird abgesaugt und mit Acetonitril und Diethylether gewaschen. Ausbeute: 2.65 g (54%, Hydrochlorid); Schmelzbereich: 220°C (Zersetzung).

### b) 1-{3-[2-(6-Benzyloxy-3-oxo-3,4-dihydro-2H-benzo[1,4]oxazin-8-yl)-2-hydroxy-ethylamino]-3-methyl-butyl }-1,4-dihydro-benzo[ 1,3]oxazin-2-on

357 mg (1 mmol) 6-Benzyloxy-8-(2-ethoxy-2-hydroxy-acetyl)-4H-benzo[1,4]oxazin-3-on und 234 mg (1 mmol) 1-(3-Amino-3-methyl-butyl)-1,4-dihydro-benzo[1,3]oxazin-2-on in 5 mL Tetrahydrofuran werden 15 Minuten bei 60°C gerührt. Man kühlt auf 0°C ab und tropft unter Argonatmosphäre 1.5 mL einer 2 molaren Lösung von Lithiumborhydrid in Tetrahydrofuran zu. Es wird 15 min bei 0°C gerührt, mit 10 mL Dichlormethan und 3 mL Wasser versetzt, eine weitere Stunde gerührt und dann über Kieselgur filtriert. Man eluiert mit Dichlormethan und destilliert die Lösungsmittel ab. Der Rückstand wird mittels präparativer HPLC (Reverse Phase, Acetonitril/Wasser-Gradient mit 0.1% Trifluoressigsäure) gereinigt. Ausbeute: 196 mg (30%, Trifluoracetat); Massenspektroskopie: [M]⁺ = 532.

### c) 1-{3-[2-(6-Benzyloxy-3-oxo-3,4-dihydro-2H-benzo[1,4]oxazin-8-yl)-2-hydroxy-ethylamino]-3-methyl-butyl}-1,4-dihydro-benzo[1,3]oxazin-2-on

196 mg (0.3 mmol) 1-{3-[2-(6-Benzyloxy-3-oxo-3,4-dihydro-2H-benzo[1,4]oxazin-8-yl)-2-hydroxy-ethylamino]-3-methyl-butyl}-1,4-dihydro-benzo[1,3]oxazin-2-on werden in 5 Ethanol gelöst und mit Palladium auf Kohle (10%ig) als Katalysator bei 3 bar und Raumtemperatur hydriert. Der Katalysator wird abgetrennt und das Rohprodukt in Acetonitril/Diethylether auskristallisiert. Ausbeute: 48 mg (29%, Trifluorethylacetat); Massenspektroskopie: [M+H]⁺ = 442.

### Beispiel 6: 4-Ethyl-1-{3-[2-hydroxy-2-(6-hydroxy-3-oxo-3,4-dihydro-2H-benzo[1,4]oxazin-8-yl)-ethylamino]-3-methyl-butyl }-1,4-dihydro-benzo[1,3]oxazin-2-on

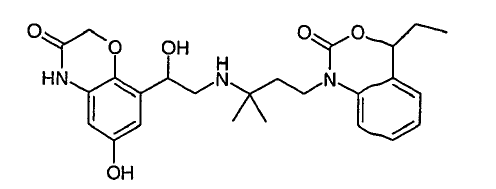

### a) 1-(3-Amino-3-methyl-butyl)-4-ethyl-1 4-dihydro-benzo[1,4]oxazin

Eine Lösung von 17.7 g (0.10 mol) 4-Ethyl-1,4-dihydro-benzo[1,3]oxazin-2-on in 85 mL HMPT wird mit 4.8 g Natriumhydrid (55-60%) versetzt und langsam auf 60°C erwärmt. Nach beendeter Wasserstoffentwicklung wird noch 30 min bei 80°C gerührt und dann auf Raumtemperatur abgekühlt. 25 g (0.12 mol) (3-Chlor-1,1-dimethyl-propyl)-(1-phenyl-methyliden)-amin, gelöst in 25 mL HMPT, werden zugegeben und man läßt drei Stunden bei 100°C rühren. Die Reaktionsmischung wird abgekühlt, auf Eiswasser gegossen und mit Ethylacetat extrahiert. Die vereinigten organischen Phasen werden mit Wasser gewaschen, mit Natriumsulfat getrocknet und eingeengt. Der Rückstand- wird zusammen mit 240 mL IN Salzsäure auf 60°C erwärmt und nach Abkühlung mit Diethylether extrahiert. Die wässrige Phase wird mit konz. Natronlauge alkalisch gestellt und mit Ethylacetat extrahiert. Die vereinigten organischen Phasen werden mit Natriumsulfat getrocknet und eingeengt. Der Rückstand wird unter Erwärmung in Ethylac etat gelöst, mit einer äquimolaren Mengen Maleinsäure versetzt und langsam abgekühlt. Der ausfallende Niederschlag wird abgesaugt, mit Ethylacetat gewaschen und getrocknet. Ausbeute: 26.1 g (69%, Maleat); Schmelzbereich: 134°C.

### b) 1-{3-[2-(6-Benzyloxy-3-oxo-3 4-dihydro-2H-benzo[1,4]oxazin-8-yl)-2-hydroxy-ethylaminol-3-methyl-butyl }-4-ethyl-1,4-dihydro-benzo[1,3]oxazin-2-on

357 mg (1 mmol) 6-Benzyloxy-8-(2-ethoxy-2-hydroxy-acetyl)-4H-benzo[1,4]oxazin-3-on und 530 mg (1 mmol) 1-(3-Amino-3-methyl-butyl)-4-ethyl-1,4-dihydro-benzo[1,4]oxazin werden in Analogie zur Vorschrift 5b) umgesetzt und aufgearbeitet.
Ausbeute: 308 mg (46%, Trifluoracetat); Massenspektroskopie: [M]⁺ = 560.

### c) 4-Ethyl-1-{3-[2-hydroxy-2-(6-hydroxy-3-oxo-3,4-dihydro-2H-benzo[1,4]oxazin-8-yl)-ethylamino]-3-methyl-butyl}-1,4-dihydro-benzo[1,3]oxazin-2-on

308 mg (0.46 mmol) 1-{3-[2-(6-Benzyloxy-3-oxo-3,4-dihydro-2H-benzo[1,4]oxazin-8-yl)-2-hydroxy-ethylamino]-3-methyl-butyl}-4-ethyl-1,4-dihydro-benzo[1,3]oxazin-2-on werden mit Palladium auf Kohle (10%ig) als Katalysator bei Raumtemperatur und 3 bar Wasserstoffdruck hydriert. Der Katalysator wird abgetrennt, das Filtrat eingeengt und der Rückstand chromatographiert (Reverse Phase; Acetonitril/Wasser-Gradient). Ausbeute: 14 mg (5%, Trifluoracetat); Massenspektroskopie: [M]⁺ = 470.

### Beispiel 7: 8-{2-[1,1-Dimethyl-3-(2-oxo-3,4-dihydro-2H-chinolin-1-yl)-propylamino]-1-hydroxy-ethyl}-6-hydroxy-4H-benzo[1,4]oxazin-3-on

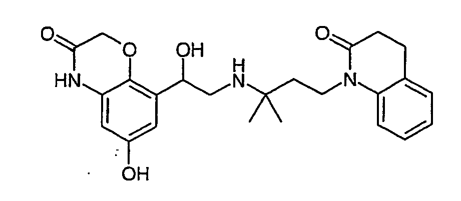

### a) 1-(3-Amino-3-methyl-butyl)-3,4-dihydro-chinolin-2-on

Die Herstellung erfolgt in Analogie zu dem für Beispiel 6a) beschriebenen Verfahren aus 15.7 g (107 mmol) 3,4-Dihydro-chinolin-2-on und 24.9 g (119 mmol) (3-Chlor-1,1-dimethyl-propyl)-(1-phenyl-methyliden)-amin. In Abweichung zur vorstehend genannten Vorschrift wird das Produkt nicht als Maleat, sondern als Hydrochlorid ausgefällt. Ausbeute: 6.9 g (24%, Hydrochlorid); Schmelzbereich: 200-203°C.

### b) 8-{2-[1,1-Dimethyl-3-(2-oxo-3,4-dihydro-2H-chinolin-1-yl)-propylamino]-1-hydroxyethyl}-6-hydroxy-4H-benzo[1,4]oxazin-3-on

Herstellung aus 357 mg (1 mmol) 6-Benzyloxy-8-(2-ethoxy-2-hydroxy-acetyl)-4H-benzo[1,4]oxazin-3-on und 232 mg (1 mmol) 1-(3-Amino-3-methyl-butyl)-3,4-dihydro-chinolin-2-on in Analogie zu dem für Beispiel 5c) und beschriebenen Verfahren. Die abschließende Reinigung des Produkts erfolgt mittels präparativer HPLC (Reverse Phase, Acetonitril/Wasser-Gradient mit 0.1% Trifluoressigsäure). Ausbeute: 94 mg (17%, Trifluoracetat); Massenspektroskopie: [M]⁺= 440.

### Beispiel 8: 4,4-Diethyl-1-{3-[2-hydroxy-2-(8-hydroxy-2-oxo-1,2-dihydro-chinolin-5-yl)-ethylamino]-3-methyl-butyl }-1,4-dihydro-benzo[1,3]oxazin-2-on

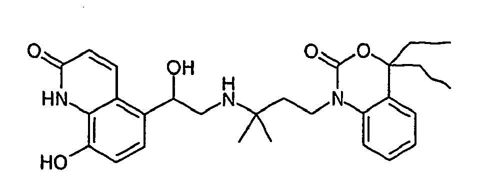

### a) 1-{3-[2-(8-Benzyloxy-2-oxo-1,2-dihydro-chinolin-5-yl)-2-hydroxy-ethylamino]-3-methyl-butyl}-4,4-diethyl-1,4-dihydro-benzo[1,3]oxazin-2-on

400 mg (1.4 mmol) 8-Benzyloxy-5-oxiranyl-chinolin-2-on und 436 mg (1.5 mmol) 1-(3-Amino-3-methyl-butyl)-4,4-diethyl-1,4-dihydro-benzo[1,3]oxazin-2-on in 5 mL n-Butanol werden 6 Stunden bei 140°C gerührt. Das Lösungsmittel wird abdestilliert und der Rückstand chromatographisch (Reverse Phase; Acetonitril/Wasser-Gradient) gereinigt. Beigefarbener Feststoff. Ausbeute: 160 mg (20%); Massenspektroskopie: [M]⁺ = 584.

### b) 4,4-Diethyl-1-{3-[2-hydroxy-2-(8-hydroxy-2-oxo-1,2-dihydro-chinolin-5-yl)-ethylamino]-3-methyl-butyl}-1,4-dihydro-benzo[1,3]oxazin-2-on

160 mg (0.3 mmol) 1-{ 3-[2-(8-Benzyloxy-2-oxo-1,2-dihydro-chi nolin-5-yl)-2-hydroxy-ethylamino]-3-methyl-butyl}-4,4-diethyl-1,4-dihydro-benzo[1,3] oxazin-2-on werden in 5 mL Methanol gelöst und in Gegenwart von Palladium auf Kohle (10%) hydriert. Ausbeute: 49 mg (34%); Massenspektroskopie: [M]⁺ = 494.

### Beispiel 9: 4,4-Diethyl-1-{3-[2-hydroxy-2,2-(6-hydroxy-2,2-dimethyl-3-oxo-3,4-dihydro-2H-benzo[1,4]oxazin-8-yl)-ethylamino]-3-methyl-butyl)-1,4-dihydro-benzo[d][1,3]oxazin-2-on

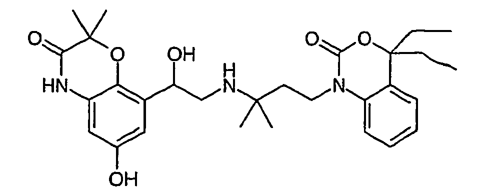

### a) N-(3-Acetyl-5-benzyloxy-2-hydroxy-phenyl)-2-brom-2-methy]-propionamid

Zu einer Lösung von 5.15 g (20 mmol) 1-(3-Amino-5-benzyloxy-2-hydroxy-phenyl)-ethanon in 20 mL Pyridin werden bei 5-20°C 4.64 g (25 mmol) 2-Brom-2-methyl-propionylchlorid getropft. Nach beendeter Zugabe wird 15 Minuten gerührt, mit Eiswasser und 100 mL Ethylacetat versetzt und mit konz. Salzsäure angesäuert. Die organische Phase wird abgetrennt, mit Wasser gewaschen und mit Natriumsulfat getrocknet. Nach dem Abdestillieren des Lösungsmittels wird der Rückstand aus einem Diethylether/Petrolether-Gemisch kristallisiert. Ausbeute: 6.8 g (84%); Schmelzbereich: 88-90°C.

### b) 8-Acetyl-6-benzyloxy-2,2-dimethyl-4H-benzo[1,4]oxazin-3-on

6.60 g (16.2 mmol) N-(3-Acetyl-5-benzyloxy-2-hydroxy-phenyl)-2-brom-2-methyl-propionamid und 2.76 g (20 mmol) Kaliumcarbonat werden 1 Stunde in 70 mL Acetonitril unter Rückfluß gerührt. Der Feststoff wird abgesaugt, das Filtrat eingeengt und der Rückstand mit 30 mL Ethylacetat versetzt. Nach erneuter Filtration und dem Abdestillieren des Lösungsmittels wird das Rohprodukt aus wenig Methanol kristallisiert. Ausbeute: 1.00 g (19%); Massenspektroskopie [M+H]⁺ = 326; Schmelzbereich = 148-150°C.

### c) 6-Benzyloxy-8-(2-ethoxy-2-hydroxy-acetyl)-2,2-dimethyl-benzo[1,4]oxazin-3-on

50.12 g (154 mmol) 8-Acetyl-6-benzyloxy-2,2-dimethyl-benzo[1,4]oxazin-3-on werden mit Selendioxid als Oxidationsmittel und Aktivkohle in refluxierendem Dioxan und etwas Wasser umgesetzt. Nach Abkühlung wird der Feststoff abfiltriert und mit Dioxan gewaschen. Das Filtrat wird eingeengt und der Rückstand in 550 mL Ethanol gelöst und für 30 Minuten unter Rückfluß erhitzt. Es wird filtriert und die Mutterlauge wird auf -18°C gekühlt, wobei ein Feststoff ausfällt, der abgesaugt wird. Nach Umkristallisation aus Ethanol liegt das Produkt in Form eines beigefarbenen Feststoffs vor. Ausbeute: 8.95 g (15%).

### d) 1-{3-[2-(6-Benzyloxy-2,2-dimethyl-3-oxo-3,4-dihydro-2H-benzo[1,4]oxazin-8-yl)-2-hydroxy-ethylaminol]-3-methyl-butyl}-4,4-diethyl-1,4-dihydro-benzo[d][1,3]oxazin-2-on

Herstellung aus 406 mg (1 mmol) 6-Benzyloxy-8-(2-ethoxy-2-hydroxy-acetyl)-2,2-dimethyl-benzo[1,4]oxazin-3-on und 290 mg (1 mmol) 1-(3-Amino-3-methyl-butyl)-4,4-diethyl-1,4-dihydro-benzo[1,3]oxazin-2-on analog zu dem für Beispiel 5b) beschriebenen Verfahren. Die Zielverbindung wird mittels Chromatographie an einer kurzen Säule mit Kieselgel (Dichlormethan/Methanol-Gradient) gereinigt. Weißer Feststoff. Ausbeute: 145 mg (24%); Massenspektroskopie [M+H]⁺= 616.

### e) 4,4-Diethyl-1-{3-[2-hydroxy-2-(6-hydroxy-2,2-dimethyl-3-oxo-3 4-dihydro-2H-benzo[1,4]oxazin-8-yl)-ethylamino]-3-methyl-butyl}-1,4-dihydro-benzo[d][1,3]oxazin-2-on

130 mg (0.21 mmol) 1-{3-[2-(6-Benzyloxy-2,2-dimethyl-3-oxo-3,4-dihydro-2H-benzo[1,4]oxazin-8-yl)-2-hydroxy-ethylamino]-3-methyl-butyl}-4,4-diethyl-1,4-dihydro-benzo[d][1,3]oxazin-2-on werden in 5 mL Methanol gelöst und in Gegenwart von Palladium auf Kohle (10%ig) bei Raumtemperatur hydriert. Der Katalysator wird abgesaugt, das Filtrat eingeengt und der Rückstand chromatographisch gereinigt (Reverse Phase; Acetonitril/Wasser-Gradient). Weißer Feststoff. Ausbeute: 41 mg (37%); Massenspektroskopie [M+H]⁺ = 526.

### Beispiel 10: 4,4-Diethyl-1-{3-[2-hydroxy-2-(5-hydroxy-2-oxo-2,3-dihydro-benzooxazol-7-yl)-ethylamino]-3-methyl-butyl}-1,4-dihydro-benzo[d][1,3]oxazin-2-on

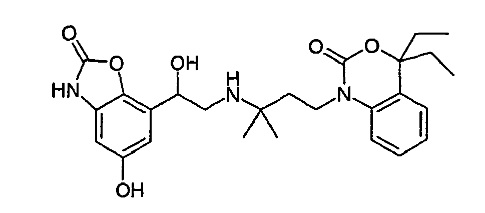

### a) 7-Acetyl-5-benzyloxy-3H-benzooxazol-2-on

Zu einer Lösung von 22.7 g (88.22 mmol) 1-(3-Amino-5-benzyloxy-2-hydroxy-phenyl)-ethanon in Toluol (200 mL) werden bei 0 °C 51.1 mL (97.04 mmol) einer Phosgen-Lösung (20 gew.% in Toluol) zugegeben. Anschließend werden 30.7 mL (220.6 mmol) Triethylamin so zugetropft, dass die Temperatur 5 °C nicht überschreitet. Nach 1 h Rühren bei Raumtemperatur werden weitere 4.6 mL Phosgen-Lösung und 12 mL Triethylamin bei 0 °C zugegeben. Es wird 1 h bei Raumtemperatur gerührt, mit Dichlormethan verdünnt und mit gesättigter wässriger Ammoniumchlorid-Lösung (500 mL) und 2 N wässriger Salzsäure (10 mL) versetzt. Nach dem Abtrennen der wässrigen Phase wird diese erschöpfend mit Dichlormethan extrahiert. Die vereinigten organischen Phasen werden mit Wasser und gesättigter wässriger Natriumchlorid-Lösung gewaschen, mit Natriumsulfat getrocknet und i. vac. eingeengt, wobei sich ein beiger Feststoff ausfällt. Der Niederschlag wird abfiltriert, mit wenig Toluol gewaschen und bei 50 °C i. vac. getrocknet.
Ausbeute: 18.5 g (74%); R_{f}= 0.19 (Kieselgel, Toluol/Aceton 95:10); ESI-MS: [M+H]⁺= 284.

### b) 5-Benzyloxy-7-(2-ethoxy-2-hydroxy-acetyl)-3H-benzooxazol-2-on

Zu einer Lösung von 12.0 g (42.4 mmol) 7-Acetyl-5-benzyloxy-3H-benzooxazol-2-on in DMSO (60 mL) werden 14.4 mL (127.1 mmol) HBr (48% in Wasser) zugegeben. Das Gemisch wird unter einem leichten Stickstoffstrom 6 h bei 60 °C gerührt, auf 600 mL Eiswasser gegossen und 20 Min. gerührt. Der gebildete Niederschlag wird abfiltriert und mit Eiswasser und kalter Wasser/Ethylacetat-Lösung (1:1) gewaschen. Der Niederschlag wird in 300 mL Ethanol und 100 mL Ethylacetat gelöst und i. vac. eingeengt. Die Prozedur wird mit 500 mL Toluol und dann mit 500 mL Ethanol wiederholt. Der Rückstand wird anschließend in 250 mL Ethanol gelöst und 1 h unter Rückfluß erhitzt. Nach dem Abdestillieren von 30 mL Ethanol wird das Gemisch auf Raumtemperatur und dann auf 0°C gekühlt. Der gebildete Niederschlag wird abfiltriert, mit 80 mL eiskaltem Ethanol und 200 mL Ether gewaschen und bei 50 °C i. vac. getrocknet. Ausbeute: 6.5 g (45%); R_{f} = 0.23 (Kieselgel, Dichlormethan/MeOH 25:2); ESI-MS: [M+H-CO₂Et]⁺ = 270.

### c) 1-{3-[2-(5-Benzyloxy-2-oxo-2,3-dihydro-benzooxazol-7-yl)-2-hydroxy-ethylamino]-3-methyl-butyl}-4,4-diethyl-1,4-dihydro-benzo[d][1,3]oxazin-2-on

Erhalten aus 343 mg (1 mmol) 5-Benzyloxy-7-(2-ethoxy-2-hydroxy-acetyl)-3*H-*benzooxazol-2-on und 290 mg (1 mmol) 1-(3-Amino-3-methyl-butyl)-4,4-diethyl-1,4-dihydro-benzo[1,3]oxazin-2-on nach dem für Beispiel 5b) beschriebenen Verfahren. Weißer Feststoff. Ausbeute: 160 mg (28%); Massenspektroskopie [M-H]⁺ = 572.

### d) 4,4-Diethyl-1-{3-[2-hydroxy-2-(5-hydroxy-2-oxo-2,3-dihydro-benzooxazol-7-yl)-ethylamino]-3-methyl-butyl}-1,4-dihydro-benzo[d][1,3]oxazin-2-on

150 mg (0.26 mmol) 1-{3-[2-(5-Benzyloxy-2-oxo-2,3-dihydro-benzooxazol-7-yl)-2-hydroxy-ethylamino]-3-methyl-butyl}-4,4-diethyl-1,4-dihydro-benzo[d][1,3]oxazin-2-on werden in 5 mL Methanol gelöst und mit Palladium auf Kohle als Katalysator 3 Stunde bei Raumtemperatur hydriert. Der Katalysator wird abgetrennt und das Filtrat eingeengt. Beigefarbener Feststoff. Ausbeute: 116 mg (92%); Massenspektroskopie [M-H]⁺ = 484.

HPLC-Methode (Methode A): Symmetry C18 (Waters); 3.5 *µ*m; 4.6 x 150 mm; Säulentemperatur: 20°C; Gradient Acetonitril/Phosphat-Puffer (pH 7) 20:80 → 80:20 in 30 Min., Fluss: 1.0 mL / min; Detektion bei 220 und 254 nm.

### Beispiel 11:4,4-Diethyl-1-{3-[2-hydroxy-2-(7-hydroxy-2-oxo-1,2-dihydro-quinolin-5-yl)-ethylamino]-3-methyl-butyl}-1,4-dihydro-benzo[d][1,3]oxazin-2-on

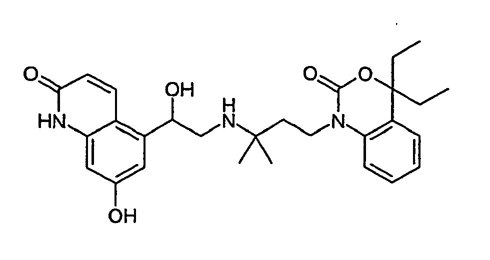

### a) Trifluormethansulfonsäure-2-acetyl-4-benzyloxy-6-nitro-phenylester

Zu einer Lösung von 1-(5-Benzyloxy-2-hydroxy-3-nitro-phenyl)-ethanon (90.0 g, 0.313 mol) in abs. Dichlormethan (940 mL) wird bei -10 °C innerhalb von 10 Min. Triethylamin (92.7 mL, 0.660 mol) zugegeben. Zu dieser roten Lösung wird innerhalb von 15 Min. eine Lösung von Trifuormethansulfonsäureanhydrid (65 mL, 0.394 mol) in abs. Dichlormethan (40 mL) zugegeben und es wird weitere 5 Min. bei -5 °C gerührt. Die braune Lösung wird mit ges. wässr. Ammoniumchlorid (400mL) und ges. wässr. NaCl (400mL) gewaschen und die Phasen werden getrennt. Trocknen mit Natriumsulfat und Einengen i. vac. liefert das Rohprodukt als Öl, das beim Stehen fest wird. Das Rohprodukt wird in Ether (150 mL) gelöst, die Lösung mit Hexan (800 mL) versetzt und der gebildete Niederschlag abfiltriert. Der Feststoff wird mit Ether/Hexan (80/20, 100 mL) verrührt, abfiltriert und im Ofen bei 40 °C getrocknet. Ausbeute: 118 g (90%); ESI-MS: [M+H]⁺ = 420.

### b) 3-(2-Acetyl-4-benzyloxy-6-nitrophenyl)-acrylsäuremethylester

Zu einer Lösung von Trifluormethansulfonsäure-2-acetyl-4-benzyloxy-6-nitro-phenylester (100.0 g, 0.238 mol) in Dioxan (360 mL) werden unter einer Stickstoffatmosphäre 100 g Molekularsieb (4 Ä), Tris(dibenzylidenaceton)dipalladium (5.88 g, 6.42 mmol), Tri-*tert-*butylphophonium-tetrafluorborat (3.50 g, 12.06 mmol), Dicyclohexylmethylamin (81.2 mL, 0.371 mol), getrocknetes Tetrabutylammoniumiodid (105.8 g, 0.286 mol) und Methylacrylat (32.6 mL, 0.362 mol) zugegeben. Das Reaktionsgemisch wird 2 Stunden bei 80 °C gerührt, mit Ether (2 L) verdünnt und mit 500 g Kieselgel versetzt. Die Suspension wird 10 Min. gerührt, filtriert und das Kieselgel mehrmals mit Ether gewaschen (4 x 600 mL). Die vereinigten organischen Phasen werden mit 1 M wässriger Salzsäure (300 mL), Natriumbicarbonat-Lösung und Natriumchlorid-Lösung gewaschen, mit Natriumsulfat getrocknet und eingeengt. Das ölige Rohprodukt wird aus heißem Ethanol (0.75 L) umkristallisiert. Der Niederschlag wird abfiltriert, mit Ethanol gewaschen (2 x 50 mL) und bei 40 °C getrocknet. Ausbeute: 32.2 g (38%); Massenspektroskopie: [M+M]⁺ = 356.

### c) 5-Acetyl-7-benzyloxy-3,4-dihydro-1H-chinolin-2-on

Eine Suspension von 3-(2-Acetyl-4-benzyloxy-6-nitrophenyl)-acrylsäure-methylester (5.0 g, 14.07 mmol) in Ethanol (100 mL) wird mit Raney-Nickel (3 g) bei Raumtemperatur und 4 bar Wasserstoffdruck hydriert. Nach 6 Stunden wird erneut Raney-Nickel (2 g) zugegeben und das Gemisch weitere 2 Stunden hydriert. Der Katalysator wird abgetrennt und das Filtrat mit 2 M wässriger Salzsäure (15 mL) versetzt. Das auskristallisierende Produkt wird abfiltriert und getrocknet. Ausbeute: 1.0 g (24%); Massenspektroskopie: [M+H]⁺ = 296.

### d) 5-Acetyl-7-benzyloxy-1H-chinolin-2-on

Zu einer Suspension von 5-Acetyl-7-benzyloxy-3,4-dihydro-1H-chinolin-2-on (13.0 g, 44.02 mmol) in Dioxan (130 mL) wird DDQ (15.0 g, 66.08 mmol) zugegeben und es wird 30 Minuten unter Rückfluß erhitzt. Das Reaktionsgemisch wird auf Raumtemperatur gekühlt und weitere 2 Stunden gerührt. Der gebildete Niederschlag wird abfiltriert, mit Dioxan (2 x 20 mL) gewaschen und in Dichlormethan/Methanol (9:1, 600 mL) gelöst. Die organische Phase wird mit Natriumbicarbonat-Lösung (2 x 100 mL) gewaschen, mit Natriumsulfat getrocknet und eingeengt. Der Rückstand wird mit Methanol verrührt, der gebildete Niederschlag abfiltriert und getrocknet. Ausbeute: 8.3 g (64%); Massenspektroskopie: [M+H]⁺ = 294.

### e) 7-Benzyloxy-5-(2-chloracetyl)-1H-chinolin-2-on

5-Acetyl-7-benzyloxy-1H-chinolin-2-on (7.0 g, 23.86 mmol) wird in einem Gemisch aus 1,2-Dichloroethan (147 mL), Eisessig (43 mL) und Wasser (7 mL) gelöst und mit N-Benzyl-trimethylammonium-dichloriodat (19.0 g, 54.58 mmol) versetzt. Das Gemisch wird 4.5 Stunden bei 65 °C gerührt, anschließend mit Natriumbicarbonat-Lösung und 5%iger. Natriumbisulfit-Lösung verdünnt und 5 Minuten gerührt. Der gebildete Niederschlag wird abfiltriert, mit Wasser (2 x 20 mL) gewaschen und im Ofen getrocknet. Ausbeute: 6.0 g (77%); Massenspektroskopie: [M+H]⁺ = 328.

### f) 7-Benzyloxy-5-oxiranyl-1H-chinolin-2-on

Zu einer Suspension von 7-Benzyloxy-5-(2-chloroacetyl)-1H-chinolin-2-on (6 g, 18.31 mmol) in THF (150 mL) wird bei 0-5 °C Lithiumborhydrid (434 mg, 19.93 mmol) zugegeben und es wird 30 Minuten gerührt. 2.5 N Natriumhydroxid-Lösung (43 mL, 107.50 mmol) wird zugegeben und man rührt 2 Stunden bei 5-10 °C und 2.5 Stunden bei Raumtemperatur. Anschließend wird das Reaktionsgemisch langsam mit Eisessig (6.5 mL) gefolgt von halbgesättigter Natriumchlorid-Lösung (100 mL) versetzt und weitere 5 Minuten gerührt. Der gebildete Niederschlag wird abfiltriert und die wässrige Phase mit Ethylacetat/THF (1/1, 5 x 100 mL) extrahiert. Der abfiltrierte Feststoff und die organischen Phasen werden vereinigt, mit Natriumsulfat getrocknet und eingeengt. Das Rohprodukt wird mit Methanol (30 mL) verrührt und der Niederschlag abfiltriert und bei Raumtemperatur getrocknet. Ausbeute: 4.8 g (89%); Massenspektroskopie: [M+H]⁺ = 294.

### g) 1-{3-[2-(7-Benzyloxy-2-oxo-1,2-dihydro-quinolin-5-yl)-2-hydroxy-ethylamino]-3-methyl-butyl}-4-,4-diethyl-1,4-dihydro-benzo[d][1,3]oxazin-2-on

Eine Suspension von 7-Benzyloxy-5-oxiranyl-1H-chinolin-2-on (112 mg, 0.382 mmol) und 1-(3-Amino-3-methyl-butyl)-4,4-diethyl-1,4-dihydro-benzo[d][1,3]oxazin-2-on (220 mg, 0.758 mmol) in Isopropanol (1.0 mL) wird in der Mikrowelle 1 h auf 135 °C erhitzt. Das Gemisch wird mit EtOAc (10 mL) verdünnt und mit 0.5 M wässr. Weinsäure-Lösung gewaschen, wobei ein Teil des Produkts ausfällt. Die Phasen werden getrennt und die wässr. Suspension wird mit soviel MeOH versetzt, bis wieder eine klare Lösung vorliegt. Die wässr. Phase wird mit Dichlormethan extrahiert und die vereinigten org. Phasen werden über Natriumsulfat getrocknet und i. vac. eingeengt. Der Rückstand wird mit EtOAc verrührt und der Niederschlag abfiltriert und i. vac. getrocknet. Ausbeute: 152 mg (68%); HPLC-MS: Rₜ = 14.8 Min. (Methode A).

### h) 4,4-Diethyl-1-{3-[2-hydroxy-2-(7-hydroxy-2-oxo-1,2-dihydro-quinolin-5-yl)-ethylamino]-3-methyl-butyl}-1,4-dihydro-benzo[d][1,3]oxazin-2-on

Eine Suspension von 1-{3-[2-(7-Benzyloxy-2-oxo-1,2-dihydro-quinolin-5-yl)-2-hydroxy-ethylamino]-3-methyl-butyl}-4,4-diethyl-1,4-dihydro-benzo[d][1,3]oxazin-2-on (152 mg, 0.338 mmol) und Pd/C (10%) (40 mg) in MeOH (12 mL) wird bei RT und 1 bar Wasserstoffdruck 4 h hydriert. Der Katalysator wird über Celite abfiltriert und mit MeOH (5 mL) gewaschen. Die org. Phase wird eingeengt, der Rückstand mit EtOAc verrieben und der gebildete Niederschlag abfiltriert und i. vac. getrocknet. Ausbeute: 76 mg (46%); R_{f} = 0.3 (Kieselgel, Dichlormethan / MeOH / ges. wässr. Ammoniak 90:10:0.5); ESI-MS: [M+H]⁺ = 494.

Für die nachfolgenden Synthesebeispiele sind spezifische Ausgangsverbindungen erforderlich, deren Darstellung nachfolgend beschrieben wird.

### Zwischenprodukt 1: 1-(3-Amino-3-methyl-butyl)-4 4-dipropyl-1,4-dihydro-benzo[d][1,3]oxazin-2-on hydrochlorid

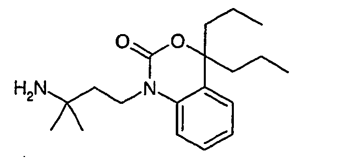

### a) 4-(2-Amino-phenyl)-heptan-4-ol

Zu einer Lösung von 7.00 mL (54.04 mmol) Anthranilsäuremethylester in abs. THF (70 mL) werden bei 0°C innerhalb von 30 Min. 90.0 mL (180.00 mmol) Propylmagnesiumchlorid (2 M in Ether) zugetropft. Das Gemisch wird 1 h bei RT gerührt und dann mit 100 mL 3 M wässr. Ammoniumchlorid-Lösung und EtOAc versetzt. Die Phasen werden getrennt und die wässr. Phase wird erschöpfend mit EtOAc extrahiert. Die vereinigten org. Phasen werden mit wässr. KHCO₃ und ges. wässr. NaCl gewaschen und mitr Natriumsulfat getrocknet. Das Rohprodukt wird ohne weitere Reinigung in dem nachfolgenden Reaktionsschritt eingesetzt. Ausbeute: 6.70 g (60%).

### b) {3-[2-1-Hydroxy-1-propyl-butyl)-phenylamino]-1,1-dimethyl-propyl}-carbaminsäure-tert-butylester

Zu einer Lösung von 3.10 g (14.05 mmol) 4-(2-Amino-phenyl)-heptan-4-ol und 3.60 g (17.88 mmol) (1,1-Dimethyl-3-oxo-propyl)-carbaminsäure-tert-butylester in MeOH (40 mL) und AcOH (6 mL) werden 1.40 g (22.27 mmol) Natriumcyanoborhydrid zugegeben. Das Gemisch wird 16 h bei RT gerührt, mit EtOAc verdünnt und mit 0.5 M wässr. KHS04 und ges. wässr. NaCl gewaschen, mit Natriumsulfat getrocknet und i. vac. eingeengt. Das Rohprodukt wird ohne weitere Reinigung in dem nachfolgenden Reaktionsschritt eingesetzt. Ausbeute: 6.00 g (quantitative Ausbeute).

### c) [1,1-Dimethyl-3-(2-oxo-4,4-dipropyl-4H benzo[d][1,3]oxazin-1-yl)-propyl]-carbaminsäure-tert-butylester

Zu einer Lösung von 6.00 g (15.28 mmol) {3-[2-(1-Hydroxy-1-propyl-butyl)-phenylamino]-1,1-dimethyl-propyl}-carbaminsäure-tert-butylester und 5.32 mL (38.21 mmol) Triethylamin in abs. THF (80 mL) werden bei 0 °C 8.85 mL (16.81 mmol) Phosgen-Lösung (20 gew.% in Toluol) langsam zugetropft. Das Gemisch wird 2 h bei RT gerührt, mit EtOAc verdünnt, mit Eis versetzt und mit ges. wässr. Ammoniak-Lösung basisch gestellt. Die wässr. Phase wird mit EtOAc erschöpfend extrahiert und die vereinigten org. Phasen werden mit ges. wässr. NaCl gewaschen, mit Natriumsulfat getrocknet und i. vac. eingeengt. Nach Säulenchromatographie (Kieselgel, Cyclohexan/EtOAc 6:1) wird das Produkt als gelbes Öl erhalten. Ausbeute: 4.57 g (71%).

### d) 1-(3-Amino-3-methyl-butyl)-4,4-dipropyl-1,4-dihydro-benzo[d][1,3]oxazin-2-on hydrochlorid

Eine Lösung von 4.20 g (10.03 mmol) [1,1-Dimethyl-3-(2-oxo-4,4-dipropyl-4*H-*benzo[*d*][1,3]oxazin-1-yl)-propyl]-carbaminsäure-tert-butylester in 35 mL Ameisensäure wird 24 h bei RT gerührt und anschließend auf Eis gegossen. Die wässr. Phase wird mit ges. wässr. Ammoniak-Lösung basisch gestellt und mit EtOAc erschöpfend extrahiert. Die vereinigten org. Extrakte werden mit ges. wässr. NaCl gewaschen, über Natriumsulfat getrocknet und i. vac. eingeengt. Der Rückstand wird in EtOAc (50 mL) aufgenommen und mit 4 mL HCl-Lösung (ges. in EtOAc) versetzt. Die Lösung wird eingedampft und zweimal mit wenig EtOH versetzt und i. vac. eingeengt. Verreiben des Rückstands mit Diisopropylether ergibt das Produkt als hygroskopisches Hydrochloridsalz. Ausbeute: 2.60 g(73%).

### Zwischenprodukt 2: 1-(3-Amino-3-methyl-butyl)-4,4-diethyl-7-fluor-1,4-dihydro-benzo[d][1,3]oxazin-2-on

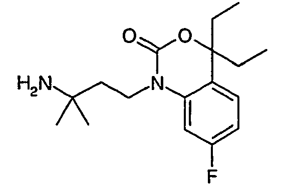

### a) 3-(2-Amino-4-fluor-phenyl)-pentan-3-ol

Das Produkt wird analog Zwischenprodukt 1a durch Reaktion von 2-Amino-4-fluor-benzoesäuremethylester und Ethylmagnesiumbromid in Dichlormethan bei - 78 °C → RT erhalten. Ausbeute: 4.1 g (99%).

### b) {3-[2-(1-Ethyl-1-hydroxy-propyl)-5-fluor-phenylaminol-1,1-dimethyl-propyl}-carbaminsäure-tert-butylester

Das Produkt wird analog Zwischenprodukt 1b ausgehend von 3-(2-Amino-4-fluor-phenyl)-pentan-3-ol und (1,1-Dimethyl-3-oxo-propyl)-carbaminsäure-tert-butylester erhalten. Das Rohprodukt wird mittels Säulenchromatographie (Kieselgel, Dichlormethan/MeOH 100:0 → 98:2) gereinigt. Ausbeute: 7.70 g (99%).

### c) [3-(4,4-Diethyl-7-fluor-2-oxo-4H-benzo[d][1,3]oxazin-1-yl)-1,1-dimethyl-propyl-carbaminsäure-tert-butylester

Das Produkt wird analog Zwischenprodukt 1c ausgehend von {3-[2-(1-Ethyl-1-hydroxypropyl)-5-fluor-phenylamino]-1,1-dimethyl-propyl}-carbaminsäure-tert-butylester erhalten. Ausbeute: 4.20 g (51 %).

### d) 1-(3-Amino-3-methyl-butyl)-4,4-diethyl-7-fluor-1,4-dihydro-benzo[d][1,3]oxazin-2-on

Das Produkt wird analog Zwischenprodukt 1d ausgehend von [3-(4,4-Diethyl-7-fluor-2-oxo-4*H*-benzo[*d*][1,3]oxazin-1-yl)-1,1-dimethyl-propyl]-carbaminsäure-tert-butylester als freie Base hergestellt. Ausbeute: 2.90 g (96%); ESI-MS: [M+H]⁺ = 309.

### Zwischenprodukt 3: 1-(3-Amino-3-methyl-butyl)-4,4-diethyl-8-methoxy-1,4-dihydro-benzo[d] [1,3]oxazin-2-on

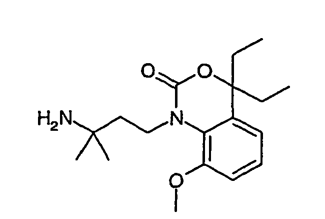

### a) 3-(2-Amino-3-methoxy-phenyl)-pentan-3-ol

Das Produkt wird analog Zwischenprodukt 1a durch Reaktion von 2-Amino-3-methoxybenzoesäuremethylester und Ethylmagnesiumbromid in Dichlormethan bei - 78 °C → RT erhalten. Ausbeute: 5.20 g (92%); HPLC-MS: Rₜ = 12.85 Min. (Methode A); ESI-MS: [M+H]⁺= 210.

### b) {3-[2-(1-Ethyl-1-hydroxy-propyl)-6-methoxy-phenylamino]-1,1-dimethyl-propyl}-carbaminsäure-tert-butylester

Das Produkt wird analog Zwischenprodukt 1b ausgehend von 3-(2-Amino-3-methoxyphenyl)-pentan-3-ol und (1,1-Dimethyl-3-oxo-propyl)-carbaminsäure-tert-butylester erhalten. Das Rohprodukt wird mittels Säulenchromatographie (Kieselgel, Cyclohexan/EtOAc 4:1) gereinigt. Ausbeute: 4.60 g (47%).

### c) [3-(4,4-Diethyl-8-methoxy-2-oxo-4H-benzo[d][1,3]oxazin-1-yl)-1,1-dimethyl-propyl]-carbaminsäure-tert-butylester

Das Produkt wird analog Zwischenprodukt 1c ausgehend von {3-[2-(1-Ethyl-1-hydroxypropyl)-6-methoxy-phenylamino]-1,1-dimethyl-propyl}-carbaminsäure-tert-butylester erhalten. Ausbeute: 4.60 g (94%).

### d) 1-(3-Amino-3-methyl-butyl)-4,4-diethyl-8-methoxy-1,4-dihydro-benzo[d][1,3]oxazin-2-on

Das Produkt wird analog Zwischenprodukt 1d ausgehend von [3-(4,4-Diethyl-8-methoxy-2-oxo-4*H*-benzo[*d*][1,3]oxazin-1-yl)-1,1-dimethyl-propyl]-carbaminsäure-tert-butylester als freie Base hergestellt. Ausbeute: 3.00 g (93%); ESI-MS: [M+H]⁺ = 321.

### Zwischenprodukt 4: 1-(3-Amino-3-methyl-butyl)-spiro(cyclohexan-1,4'-2H-3', 1'-benzoxazin)-2'-on

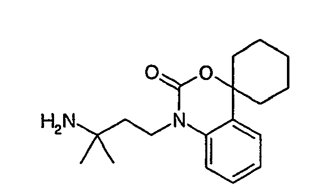

### a) 1-(2-Nitro-phenyl)-cyclohexanol

Zu einer Lösung von 20.0 g (80.32 mmol) 2-Nitro-iodbenzol in abs. THF (150 mL) werden bei -50 °C unter Stickstoffatmosphäre 40.16 mL (80.32 mmol) Phenylmagnesiumchlorid (2 M in THF) zugetropft. Nach 15 Min. Rühren werden 9.98 mL (96.30 mmol) Cyclohexanon schnell zugegeben. Das Gemisch wird auf RT erwärmt und weitere 2 h gerührt. Ges. wässr. Ammoniumchlorid-Lösung wird zugegeben und die wässr. Phase wird mit EtOAc erschöpfend extrahiert. Die vereinigten org. Extrakte werden mit ges. wässr. NaCl-Lösung gewaschen, mit Natriumsulfat getrocknet und i. vac. eingeengt. Nach Säulenchromatographie (Kieselgel, Hexan/EtOAc 20:1) wird das Produkt als bräunliches Öl erhalten. Ausbeute: 5.20 g (29%); R_{f} = 0.26 (Kieselgel, Hexan/EtOAc 10: 1); ESI-MS: [M+H-H₂O]⁺ = 204.

### b) 1-(2-Amino-phenyl)-cyclohexanol

Eine Suspension von 5.20g (16.45 mmol) 1-(2-Nitro-phenyl)-cyclohexanol und 500 mg Raney-Nickel in EtOH (70 mL) wird bei RT und 3 bar Wasserstoffdruck 4 h hydriert. Der Katalysator wird über Celite abfiltriert und das Filtrat i. vac. eingeengt. Der Rückstand wird aus Hexan umkristallisiert. Ausbeute: 1.53 g (49%); R_{f} = 0.38 (Kieselgel, Hexan/EtOAc 4:1); ESI-MS: [M+H-H₂O]⁺ = 174.

### c) {3-[2-(1-Hydroxy-cyclohexyl)-phenylamino]-1,1-dimethyl-propyl}-carbaminsäure-tert-butylester

Das Produkt wird analog Zwischenprodukt 1b ausgehend von 1-(2-Amino-phenyl)-cyclohexanol und (1,1-Dimethyl-3-oxo-propyl)-carbaminsäure-tert-butylester hergestellt. Nach Säulenchromatographie (Kieselgel, Hexan/EtOAc 7:1) wird das Produkt als farbloses Öl erhalten. Ausbeute: 2.65 g (66%); R_{f}= 0.50 (Kieselgel, Hexan/EtOAc 4:1).

### d) [3-(Spiro(cyclohexan-1,4'-2H-3',1'-b zoxazin)-2'-oxo-1-yl)-1,1-dimethyl-Propyl]-carbaminsäure-tert-butylester

Das Produkt wird analog Zwischenprodukt 1c ausgehend von {3-[2-(1-Hydroxy-cyclohexyl)-phenylamino]-1,1-dimethyl-propyl }-carbaminsäure-tert-butylester hergestellt. Ausbeute: 2.60 g (92%); R_{f}= 0.38 (Kieselgel, Hexan/EtOAc 4:1).

### e) 1-(3-Amino-3-methyl-butyl)-spiro(cyclohexan-1,4'-2H-3',1'-benzoxazin)-2'-on

Das Produkt wird analog Zwischenprodukt 1d ausgehend von [3-(Spiro(cyclohexan-1,4'-2H-3',1'-benzoxazin)-2'-oxo-1-yl)-1,1-dimethyl-propyl]-carbaminsäure-tert-butylester hergestellt. Ausbeute: 1.80 g (92%); R_{f} = 0.10 (Kieselgel, Dichlormethan/MeOH/ges. wässr. Ammoniak 95:5:0.5); ESI-MS: [M+H]⁺ = 303.

### Beispiel 12: 1-{3-[2-Hydroxy-2-(6-hydroxy-3-oxo-3,4-dihydro-2H-benzo[1,4]oxazin-8-yl)-ethylamino]-3-methyl-butyl}-4,4-dipropyl-1,4-dihydro-benzo[d][1,3]oxazin-2-on

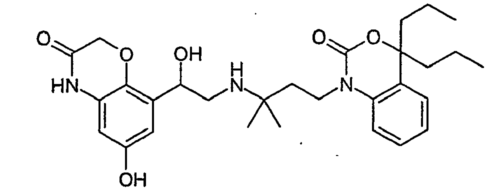

### a) 1-{3-[2-(6-Benzyloxy-3-oxo-3,4-dihydro-2H-benzo[1,4]oxazin-8-yl)-2-hydroxy-ethylamino]-3-methyl-butyl }-4,4-dipropyl-1,4-dihydro-benzo[d][1,3]oxazin-2-on

Zu einer Lösung von 200 mg (0.564 mmol) 1-(3-Amino-3-methyl-butyl)-4,4-dipropyl-1,4-dihydro-benzo[*d*][1,3]oxazin-2-on hydrochlorid in abs. THF (5 mL) werden bei RT unter einer Stickstoffatmosphäre 86 µL (0.619 mmol) Triethylamin zugegeben und es wird 30 Min. gerührt. 200 mg (0.560 mmol) 6-Benzyloxy-8-(2-ethoxy-2-hydroxy-acetyl)-4H-benzo[1,4]oxazin-3-on werden zugegeben und es wird weitere 2 h bei RT gerührt. Das Gemisch wird auf 10 °C gekühlt, mit 51 mg (2.34 mmol) Lithiumborhydrid versetzt, auf RT erwärmt und eine 1 h bei RT gerührt. Es wird erneut auf 10 °C gekühlt und langsam mit 15 mL Wasser und 20 mL Dichlormethan versetzt. Die Phasen werden getrennt und man extrahiert die wässr. Phase mit Dichlormethan. Die vereinigten org. Phasen werden mit Natriumsulfat getrocknet und i. vac. eingeengt. Der Rückstand wird in EtOAc (8 mL) gelöst und durch Zugabe von HCl-Lösung (ges. in EtOAc) auf pH 2 angesäuert. Der gebildete Niederschlag wird abfiltriert, mit EtOAc gewaschen und i. vac. getrocknet. Ausbeute: 270 mg (74%; Hydrochlorid), HPLC-MS: Rₜ = 18.7 Min. (Methode A).

### b) 1-{ 3-[2-Hydroxy-2-(6-hydroxy-3-oxo-3,4-dihydro-2H-benzo[1,4]oxazin-8-yl)-ethylamino]-3-methyl-butyl}-4,4-dipropyl-1,4-dihydro-benzo[d][1,3]oxazin-2-on

Eine Suspension von 270 mg (0.438 mmol) 1-{3-[2-(6-Benzyloxy-3-oxo-3,4-dihydro-2H-benzo[1,4]oxazin-8-yl)-2-hydroxy-ethylamino]-3-methyl-butyl }-4,4-dipropyl-1,4-dihydro-benzo[d][1,3]oxazin-2-on und 27 mg Pd/C (10%) in MeOH (8 mL) wird bei RT und 1 bar Wasserstoffdruck 3 h hydriert. Der Katalysator wird über Celite abfiltriert und mit MeOH (5 mL) gewaschen und das Filtrat i. vac. eingedampft. Der Rückstand wird in EtOAc/Dichlormethan (1:1, 10 mL) gelöst, durch Zugabe von HCl-Lösung (ges. in EtOAc) auf pH 2 angesäuert und i. vac. eingedampft. Der Rückstand wird mit Ether verrieben, filtriert und i. vac. getrocknet. Ausbeute: 80 mg (33%; Hydrochlorid), HPLC-MS: Rₜ = 12.8 Min. (Methode A), ESI-MS: [M+H]⁺= 526.

### Beispiel 13: 1-{3-[2-Hydroxy-2-(5-hydroxy-3-oxo-3,4-dihydro-2H-benzo[1,4]oxazin-8-yl)-ethylamino]-3-methyl-butyl}-4,4-dipropyl-1,4-dihydro-benzo[d][1,3]oxazin-2-on

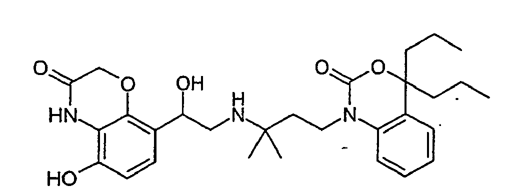

### a) 1-{3-[2-(5-Benzyloxy-3-oxo-3,4-dihydro-2H-benzo[1,4]oxazin-8-yl)-2-hydroxy-ethylamino]-3-methyl-butyl}-4,4-dipropyl-1,4-dihydro-benzo[d][1,3]oxazin-2-on

Das Produkt wird analog Beispiel 12a ausgehend von 5-Benzyloxy-8-(2,2-dihydroxy-acetyl)-4*H*-benzo[1,4]oxazin-3-on und 1-(3-Amino-3-methyl-butyl)-4,4-dipropyl-1,4-dihydro-benzo[*d*][1,3]oxazin-2-on hydrochlorid hergestellt. Das Rohprodukt wird in EtOAc gelöst, mit 5%-iger wässr. NaOH-Lösung gewaschen und mittels Säulenchromatographie (Kieselgel, Dichlormethan/MeOH 98:2 → 90:10) gereinigt. Ausbeute: 170 mg (49%); HPLC-MS: Rₜ =18.9 Min. (Methode A).

### b) 1-{3-[2-Hydroxy-2-(5-hydroxy-3-oxo-3,4-dihydro-2H-benzo[1,4]oxazin-8-yl)-ethylaminol-3-methyl-butyl}-4,4-dipropyl-1,4-dihydro-benzo[d][1,3]oxazin-2-on

Das Produkt wird analog Beispiel 12b ausgehend von 1-{3-[2-(5-Benzyloxy-3-oxo-3,4-dihydro-2H-benzo[1,4]oxazin-8-yl)-2-hydroxy-ethylamino]-3-methyl-butyl }-4,4-dipropyl-1,4-dihydro-benzo[d][1,3]oxazin-2-on hergestellt. Ausbeute: 30 mg (19%, Hydrochlorid); HPLC-MS: Rₜ = 13.0 Min. (Methode A); ESI-MS: [M+H]⁺ = 526.

### Beispiel 14: 4,4-Diethyl-7-fluor-1-{3-[2-hydroxy-2-(6-hydroxy-3-oxo-3A-dihydro-2H-benzor[1,4]oxazin-8-yl)-ethylaminol-3-methyl-butyl 1-1,4-dihydro-benzo[d][1,3]oxazin-2-on

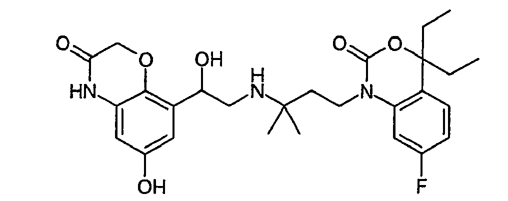

### a) 1-{3-[2-(6-Benzyloxy-3-oxo-3,4-dihydro-2H-benzo[1,4]oxazin-8-yl)-2-hydroxy-ethylamino]-3-methyl-butyl}-4,4-diethyl-7-fluor-1,4-dihydro-benzo[d][1,3]oxazin-2-on

Eine Lösung von 232 mg (0.649 mmol) 6-Benzyloxy-8-(2-ethoxy-2-hydroxy-acetyl)-4H-benzo[1,4]oxazin-3-on und 200 mg (0.649 mmol) 1-(3-Amino-3-methyl-butyl)-4,4-diethyl-7-fluor-1,4-dihydro-benzo[d][1,3]oxazin-2-on in abs. THF (5 mL) wird 2.5 h bei RT gerührt. Das Gemisch wird auf 5 °C gekühlt, mit 60 mg (2.755 mmol) Lithiumborhydrid versetzt, auf RT erwärmt und 1 h gerührt. Es wird erneut auf 5 °C gekühlt und langsam mit 15 ml Wasser und 20 mL Dichlormethan verdünnt. Die Phasen werden getrennt und die wässr. Phase wird mit Dichlormethan extrahiert. Die vereinigten org. Phasen werden mit Natriumsulfat getrocknet und i. vac. eingedampft. Der Rückstand wird mittels Säulenchromatographie (Kieselgel, Dichlormethan/MeOH 95:5) gereinigt. Ausbeute: 257 mg (65%); HPLC-MS: Rₜ = 16.5 Min. (Methode A).

### b) 4,4-Diethyl-7-fluor-1-{3-[2-hydroxy-2-(6-hydroxy-3-oxo-3,4-dihydro-2H-benzo[1,4]oxazin-8-yl)-ethylamino]-3-methyl-butyl]-1,4-dihydro-benzo-[d][1,3]oxazin-2-on

Das Produkt wird analog Beispiel 12b ausgehend von 1-{3-[2-(6-Benzyloxy-3-oxo-3,4-dihydro-2*H*-benzo[1,4]oxazin-8-yl)-2-hydroxy-ethylamino]-3-methyl-butyl}-4,4-diethyl-7-fluor-1,4-dihydro-benzo[*d*][1,3]oxazin-2-on hergestellt. Ausbeute: 170 mg (78%; Hydrochlorid); HPLC-MS: Rₜ =10.6 Min. (Methode A); ESI-MS: [M+H]⁺ = 516.

### Beispiel 15: 4,4-Diethyl-1-{3-[2-hydroxy-2-(5-hydroxy-3-oxo-3,4-dihydro-2H-benzo[1,4]oxazin-8-yl)-ethylamino]-3-methyl-butyl}-8-methoxy-1,4-dihydro-benzo[d][1,3]oxazin-2-on

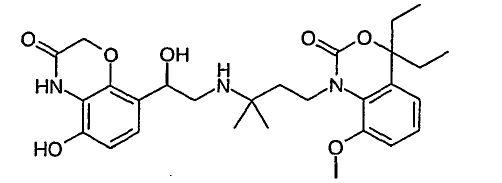

### a) 1-{3-[2-(5-Benzyloxy-3-oxo-3,4-dihydro-2H-benzo[1,4]oxazin-8-yl)-2-hydroxy-ethylamino]-3-methyl-butyl}-4,4-diethyl-8-methoxy-1,4-dihydro-benzo[d][1,3]oxazin-2-on

Das Produkt wird analog Beispiel 14a ausgehend von 5-Benzyloxy-8-(2,2-dihydroxy-acetyl)-4*H*-benzo[1,4]oxazin-3-on und 1-(3-Amino-3-methyl-butyl)-4,4-diethyl-8-methoxy-1,4-dihydro-benzo[d][1,3]oxazin-2-on hergestellt. Das Rohprodukt wird mittels Säulenchromatographie (Kieselgel, Dichlormethan/MeOH 95:5) gereinigt. Ausbeute: 70 mg (18%); HPLC-MS: Rₜ = 16.5 Min. (Methode A).

### b) 4,4-Diethyl-1-{3-[2-hydroxy-2-(5-hydroxy-3-oxo-3,4-dihydro-2H-benzo[1,4]oxazin-8-yl)-ethylamino]-3-methyl-butyl}-8-methoxy-1,4-dihydro-benzo[d][1,3]oxazin-2-on

Das Produkt wird analog Beispiel 12b ausgehend von 1-{3-[2-(5-Benzyloxy-3-oxo-3,4-dihydro-*2H-*benzo[1,4]oxazin-8-yl)-2-hydroxy-ethylamino]-3-methyl-butyl}-4,4-diethyl-8-methoxy-1,4-dihydro-benzo[*d*][1,3]oxazin-2-on erhalten. Ausbeute: 40 mg (62%); HPLC-MS: Rₜ = 13.3 Min. (Methode A); ESI-MS: [M+H]⁺ = 528.

### Beispiel 16:1-(2-{1-[2-Hydroxy-2-(5-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on

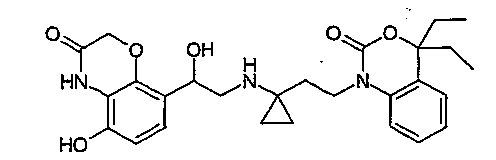

### a) 3-(4,4-Dimethyl-2-oxo-4H-benzo[d][1,3]oxazin-1-yl]-propionitril

Zu einer Lösung von 20.0 g (112 mmol) 4,4-Dimethyl-1,4-dihydro-benzo[d][1,3]oxazin-2-on und 17.4 g (126 mmol) Kaliumcarbonat in 250 mL Acetonitril werden 10.2 mL (123 mmol) Brompropionitril getropft und es wird über Nacht unter Rückfluß gerührt. Weitere 4 mL (48 mmol) Brompropionitril werden zugesetzt und es wird noch 2 Stunden unter Rückfluß gerührt. Der Feststoff wird abgesaugt, das Filtrat eingeengt und der Rückstand in Diisopropylether umkristallisiert. Weißer Feststoff. Ausbeute: 22.8 g (88%); Massenspektroskopie: [M+H]⁺ = 231.

### b) 1-[2-(1-Amino-cyclopropyl)-ethyl]-4,4-dimethyl-1,4-dihydro-benzo[d][1,3]oxazin-2-on

Eine Suspension von 6.0 g (26 mmol) 3-(4,4-Dimethyl-2-oxo-4H-benzo[d][1,3]oxazin-1-yl]-propionitril in 120 mL Diethylether wird unter Kühlung mit einem Eisbad mit 16.5 mL (56 mmol) Titan-tetra-isopropylat versetzt. Anschließend werden 18.5 mL einer 3 molaren Lösung von Ethylmagnesiumbromid in Diethylether so zugetropft, dass die Temperatur nicht über 20°C steigt. Man läßt 30 Minuten bei Raumtemperatur Rühren und gibt 7.0 mL (55 mmol) Bortrifluorid-Diethylether portionsweise unter Kühlung mit einem Eisbad zu. Es wird eine Stunde bei Raumtemperatur gerührt und unter Kühlung werden 150 mL 1 molare Natriumhydroxid-Lösung zugetropft. Das Reaktionsgemisch wird mit Diethylether verdünnt und die Phasen werden getrennt. Die wässrige Phase wird mit Diethylether extrahiert und die vereinigten organischen Phasen werden mit Natriumsulfit-Lösung und wiederholt mit 1 molarer Salzsäure ausgeschüttlet. Die Salzsäure-Phasen werden vereinigt, mit Diethylether ausgeschüttelt, mit Natriumhydroxid-Lösung alkalisch gestellt und erschöpfend mit Dichlormethan extrahiert. Die Dichlormethan-Phasen werden mit Natriumsulfat getrocknet und eingeengt. Leicht gelbes Öl. Ausbeute: 1.5 g (22%); Massenspektroskopie: [M+H]⁺ = 261.

### c) 1-(2-{1-[2-(5-Benzyloxy-3-oxo-3,4-dihydro-2H-benzo[1,4]oxazin-8-yl)-2-hydroxy-ethylamino]-cyclopropyl}-ethyl)-4,4-dimethyl-1,4-dihydro-benzo[d][1,3]oxazin-2-on

900 mg (2.5 mmol) 5-Benzyloxy-8-(2-ethoxy-2-hydroxy-acetyl)-4*H*-benzo[1,4]oxazin-3-on und 700 mg (2.7 mmol) 1-[2-(1-Amino-cyclopropyl)-ethyl]-4,4-dimethyl-1,4-dihydro-benzo[d][1,3]oxazin-2-on werden in 20 mL Ethanol gelöst und jeweils 30 Minuten bei 80°C und 50°C gerührt. Die Reaktionsmischung wird abgekühlt, mit 200 mg (5.3 mmol) Natriumborhydrid versetzt und 2 Stunden bei Raumtemperatur gerührt. Es wird Eisessig zugegeben, 10 Minuten gerührt und eingeengt. Der Rückstand wird in Dichlormethan aufgenommen und nacheinander mit Kaliumhydrogensulfat-Lösung, 15%iger Kaliumcarbonat-Lösung und Natriumhydrogencarbonat-Lösung gewaschen. Anschließend wird die organische Phase mit Natriumsulfat getrocknet und vom Lösungsmittel befreit. Der Rückstand wird säulenchromatographisch gereinigt (Kieselgel; Ethylacetat/Methanol/Ammoniak-Gradient). Umkristallisation aus Diisopropylether. Weißer Feststoff. Ausbeute: 690 mg (49%); Massenspektroskopie: [M+H]⁺ = 558.

### d) 1-(2-{1-[2-Hydroxy-2-(5-hydroxy-3-oxo-3,4-dihydro-2H-benzor[1,4]oxazin-8-yl)-ethylamino]-cyclopropyl}-ethyl)-4,4-dimethyl-1,4-dihydro-benzo[d][1,3]oxazin-2-on

650 mg (1.17 mmol) 1-(2-{1-[2-(5-Benzyloxy-3-oxo-3,4-dihydro-2H-benzo[1,4]oxazin-8-yl)-2-hydroxy-ethylamino]-cyclopropyl}-ethyl)-4,4-dimethyl-1,4-dihydro-benzo[d][1,3]oxazin-2-on werden in 30 mL Methanol gelöst, mit Palladium auf Kohle (10%ig) versetzt und bei Raumtemperatur und 3 bar Wasserstoffdruck hydriert. Ausbeute: 240 mg (44%); Massenspektroskopie: [M+H]⁺ = 468.

### Beispiel 17: 1-(2-{1-[2-Hydroxy-2-(6-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-on

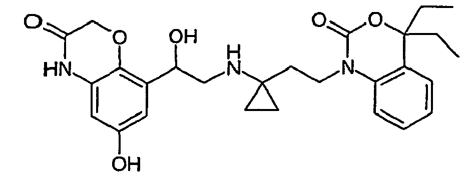

### a) 1-(2-{1-[2-(6-Benzyloxy-3-oxo-3,4-dihydro-2H-benzo[1,4]oxazin-8-yl)-2-hydroxy-ethylamino]-cyclopronyl}-ethyl)-4,4-dimethyl-1,4-dihydro-benzo[d][1,3]oxazin-2-on

Herstellung aus 900 mg (2.5 mmol) 6-Benzyloxy-8-(2-ethoxy-2-hydroxy-acetyl)-4H-benzo[1,4]oxazin-3-on und 700 mg (2.7 mmol) 1-[2-(1-Amino-cyclopropyl)-ethyl]-4,4-dimethyl-1,4-dihydro-benzo[d][1,3]oxazin-2-on in Analogie zu dem für Beispiel 16a beschriebenen Verfahren. Weißer Feststoff. Ausbeute: 630 mg (45%); Massenspektroskopie: [M+H]⁺ = 558.

### b) 1-(2-{1-[2-Hydroxy-2-(6-hydroxy-3-oxo-3,4-dihydro-2H-benzo[1,4]oxazin-8-yl)-ethylamino]-cyclopropyl}-ethyl)-4,4-dimethyl-1,4-dihydro-benzo[d][1,3]oxazin-2-o

590 mg (1.06 mmol) 1-(2-{1-[2-(6-Benzyloxy-3-oxo-3,4-dihydro-2H-benzo[1,4]oxazin-8-yl)-2-hydroxy-ethylamino]-cyclopropyl}-ethyl)-4,4-dimethyl-1,4-dihydro-benzo[d][1,3]oxazin-2-on werden in 30 mL Methanol gelöst und in Gegenwart von Palladium auf Kohle (10%ig) bei Raumtemperatur und 3 bar Wasserstoffdruck hydriert. Ausbeute: 180 mg (36%); Massenspektroskopie: [M+H]⁺ = 468.

Die nachfolgend aufgeführten Beispiele werden in Analogie zu den vorstehend beschriebenen Verfahren erhalten.

### Beispiel 18: 1-{3-[2-Hydroxy-2-(6-hydroxy-3-oxo-3.4-dihydro-2H-benzo[1,4]oxazin-8-yl)-ethylaminol-3-methyl-butyl }-spiro(cyclohexan-1,4' -2H-3',1'-benzoxazin)-2'-on

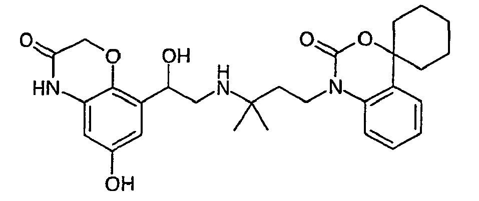

### Beispiel 19: 1-{3-[2-Hydroxy-2-(5-hydroxy-3-oxo-3,4-dihydro-2H-benzo[1,4]oxazin-8-yl)-ethylaminol-3-methyl-butyl}-spiro(cyclohexan-1,4'-2H-3',1'-benzoxazin)-2'-on

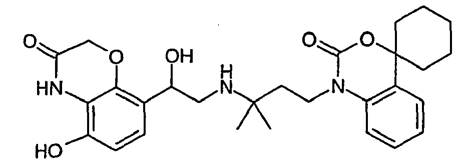

### Beispiel 20: 4,4-Dimethyl-1-{3-[2-hydroxy-2-(6-hydroxy-3-oxo-3,4-dihydro-2H-benzor[1,4]oxazin-8-yl)-ethylamino]-propyl}-1,4-dihydro-benzo[d]oxazin-2-on

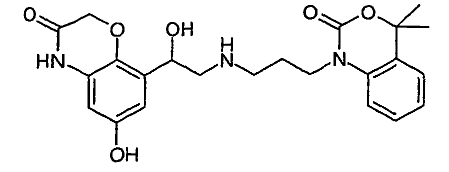

### Beispiel 21: 4,4-Dimethyl-1-{3-[2-hydroxy-2-(5-hydroxy-3-oxo-3,4-dihydro-2H-benzo[1,4]oxazin-8-yl)-ethylamino]-propyl}-1,4-dihydro-benzo[d][1,3]oxazin-2-on

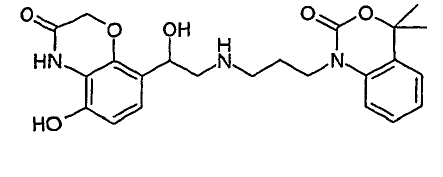

Wie gefunden wurde, zeichnen sich die Verbindungen der Formel 1 durch vielfältige Anwendungsmöglichkeiten auf therapeutischem Gebiet aus. Hervorzuheben sind solche Anwendungsmöglichkeiten, für welche die erfindungsgemäßen Verbindungen der Formel 1 aufgrund ihrer pharmazeutischen Wirksamkeit als Betamimetikum bevorzugt zur Anwendung gelangen können.
Dies sind beispielsweise die Behandlung von entzündlichen und obstruktiven Atemwegserkrankungen, bevorzugt die Therapie des Asthmas oder der COPD (chronisch obstruktive Lungenerkrankung), die Hemmung verfrüht einsetzender Wehen in der Geburtshilfe (Tokolyse), die Wiederherstellung des Sinusrhythmus im Herzen bei atrioventrikulärem Block, sowie die Behebung bradykaler Herzrhythmusstörungen (Antiarrhythmikum), die Therapie des Kreislaufschocks (Gefäßerweiterung und Steigerung des Herzzeitvolumens) sowie die Behandlung von Juckreiz und Entzündungen der Haut.

Ein Aspekt der vorliegenden Erfindung betrifft die Verwendung der Verbindungen der Formel 1 als Arzneimittel. Ein weiterer Aspekt der vorliegenden Erfindung betrifft die Verwendung der Verbindungen der Formel 1 zur Herstellung eines Arzneimittels zur Behandlung von Erkrankungen, in denen therapeutisch wirksame Dosen eines Betamimetikums einen therapeutischen Nutzen entfalten können. Besonders. bevorzugt ist dabei die Verwendung von Verbindungen der Formel 1 zur Herstellung eines Arzneimittels zur Behandlung von entzündlichen und obstruktiven Atemwegserkrankungen, besonders bevorzugt von Asthma oder COPD, zur Hemmung verfrüht einsetzender Wehen in der Geburtshilfe (Tokolyse), zur Wiederherstellung des Sinusrhythmus im Herzen bei atrioventrikulärem Block, zur Behebung bradykaler Herzrhythmusstörungen, zur Therapie des Kreislaufschocks (Gefäßerweiterung und Steigerung des Herzzeitvolumens) sowie zur Behandlung von Juckreiz und Entzündungen der Haut. Besonders bevorzugt ist erfindungsgemäß die Verwendung von Verbindungen der Formel 1 zur Herstellung eines Arzneimittels zur Behandlung von entzündlichen und obstruktiven Atemwegserkrankungen, besonders bevorzugt zur Behandlung von Asthma oder COPD. Von besonderer Bedeutung ist ferner die vorstehend genannte Verwendung von Verbindungen der Formel **1** zur Herstellung eines Arzneimittels zur einmal täglichen Behandlung von entzündlichen und obstruktiven Atemwegserkrankungen, besonders bevorzugt zur einmal täglichen Behandlung von Asthma oder COPD.

Geeignete Anwendungsformen zur Applikation der Verbindungen der Formel 1 sind beispielsweise Tabletten, Kapseln, Zäpfchen, Lösungen, Pulver etc. Der Anteil der pharmazeutisch wirksamen Verbindung(en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.
Säfte der erfindungsgemäßen Wirkstoffe können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid; oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösungsmittel als Lösevermittler bzw. Hilfslösungsmittel eingesetzt werden können, hergestellt und in Injektionsflaschen oder Ampullen oder Infusionsflaschen abgefüllt.

Die eine oder mehrere Wirkstoffe enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösungsmittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Bei der erfindungsgemäß bevorzugten Applikation der Verbindungen der Formel 1 zur Therapie von Asthma oder COPD werden besonders bevorzugt inhalativ applizierbare Darreichungsformen bzw. pharmazeutische Formulierungen eingesetzt. Als inhalierbare Darreichungsformen kommen Inhalationspulver, treibgashaltige Dosieraerosole oder treibgasfreie Inhalationslösungen in Betracht. Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

Erfindungsgemäß einsetzbare Inhalationspulver können 1 entweder allein oder im Gemisch mit geeigneten physiologisch unbedenkliche Hilfsstoffen enthalten. Sind die Wirkstoffe 1 im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Die Hilfsstoffe weisen im Rahmen der erfindungsgemäßen Inhalationspulver eine maximale mittlere Teilchengröße von bis zu 250µm, bevorzugt zwischen 10 und 150µm, besonders bevorzugt zwischen 15 und 80µm auf. Gegebenenfalls kann es sinnvoll erscheinen, den vorstehend genannten Hilfsstoffen feinere Hilfsstofffraktionen mit einer mittleren Teilchengröße von 1 bis 9µm beizumischen. Letztgenannte feinere Hilfsstoffe sind ebenfalls ausgewählt aus der vorstehend genannten Gruppe an einsetzbaren Hilfsstoffen. Schließlich wird zur Herstellung der erfindungsgemäßen Inhalationspulver mikronisierter Wirkstoff 1, vorzugsweise mit einer mittleren Teilchengröße von 0,5 bis 10µm, besonders bevorzugt von 1 bis 5µm, der Hilfsstoffmischung beigemischt. Verfahren zur Herstellung der erfindungsgemäßen Inhalationspulver durch Mahlen und Mikronisieren sowie durch abschließendes Mischen der Bestandteile sind aus dem Stand der Technik bekannt. Die erfindungsgemäßen Inhalationspulver können mittels aus dem Stand der Technik bekannten Inhalatoren appliziert werden.

Erfindungsgemäße treibgashaltige Inhalationsaerosole können 1 im Treibgas gelöst oder in dispergierter Form enthalten. Hierbei können 1 in getrennten Darreichungsformen oder in einer gemeinsamen Darreichungsform enthalten sein, wobei 1 entweder beide gelöst, beide dispergiert oder jeweils nur eine Komponente gelöst und die andere dispergiert enthalten sein können.
Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind halogenierte Alkanderivate ausgewählt aus TG134a und TG227 und Mischungen derselben.

Die treibgashaltigen Inhalationsaerosole können ferner weitere Bestandteile wie Kosolventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt.

Die vorstehend genannten treibgashaltigen Inhalationaerosole können mittels im Stand der Technik bekannten Inhalatoren (MDIs = metered dose inhalers) appliziert werden.

Ferner kann die Applikation der erfindungsgemäßen Wirkstoffe 1 in Form von treibgasfreien Inhalationslösungen und Inhalationssuspensionen erfolgen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis 70 Volumenprozent, insbesondere bei bis zu 60 Volumenprozent und besonders bevorzugt bei bis zu 30 Volumenprozent. Die restlichen Volumenprozente werden von Wasser aufgefüllt. Die 1 enthaltenden Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.
In diesen Formulierungen kann gegebenenfalls auf den Zusatz von Editinsäure (EDTA) oder einem der bekannten Salze davon, Natriumedetat, als Stabilisator oder Komplexbildner verzichtet werden. Andere Ausführungsformen beinhalten diese Verbindung(en). In einer solchen bevorzugten Ausführungsform liegt der Gehalt bezogen auf Natriumedetat unter 100 mg / 100 ml, bevorzugt unter 50 mg/ 100ml, besonders bevorzugt unter 20 mg/ 100ml. Generell sind solche Inhalationslösungen bevorzugt, in denen der Gehalt an Natriumedetat bei 0 bis 10mg/100ml liegt.
Den treibgasfreien Inhalationslösungen können Co-Solventien und/oder weitere Hilfsstoffe zugesetzt werden. Bevorzugte Co-Solventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon sonstige Stabilisatoren, Komplexbildner, Antioxidantien und/oder Konservierungsstoffe, die die Verwendungsdauer der fertigen Arzneimittelformulierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien.
Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.
Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration. Die vorstehend genannten Konservierungsstoffe sind vorzugsweise in Konzentrationen von bis zu 50mg/100ml, besonders bevorzugt zwischen 5 und 20 mg/100ml enthalten.
Bevorzugte Formulierungen enthalten außer dem Lösungsmittel Wasser und dem Wirkstoff 1 nur noch Benzalkoniumchlorid und Natriumedetat.
In einer anderen bevorzugten Ausführungsform wird auf Natriumedetat verzichtet.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Milligrammbereich liegen.

Ein weiterer Aspekt der vorliegenden Erfindung betrifft die vorstehend genannten pharmazeutische Formulierungen als solche, die durch einen Gehalt an einer Verbindung der Formel **1** gekennzeichnet sind, besonders bevorzugt die vorstehend genannten inhalativ applizierbaren pharmazeutischen Formulierungen.

Die nachfolgenden Formulierungsbeipiele illustrieren die vorliegende Erfindung ohne sie jedoch in ihrem Umfang zu beschränken:

| A) | Tabletten | pro Tablette |
|---|---|---|
| | Wirkstoff der Formel **1** | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette |
|---|---|---|
| | Wirkstoff der Formel **1** | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpresst das Gemisch zu Tabletten geeigneter Größe.

| C) | Ampullenlösung | |
|---|---|---|
| | Wirkstoff der Formel **1** | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff der Formel **1** | 0,005 |
| | Sorbitantrioleat | 0,1 |
| | Monofluortrichlormethan und | |
| | TG134a : TG227 2:1 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| | | |
|---|---|---|
| F) | Inhalationpulver | |
| | Wirkstoff der Formel **1** | 12 µg |
| | Lactose Monohydrat | ad 10 mg |

Die Herstellung des Inhalationspulvers erfolgt in üblicher Art und Weise durch Mischen der einzelnen Bestandteile.

## Patentansprüche

1. Verbindungen der Formel 1 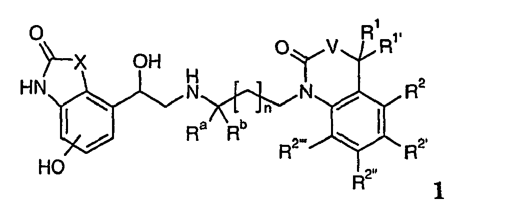 worin
X ein Gruppe -O-, -NH-, -CH₂-O-, -CHMe-O-, -C(Me)₂-O-, -CH₂-NH-, -CHMe-NH-, -C(Me)₂-NH-, -CH=CH- oder -CH₂-CH₂- bedeutet;
V eine zweibindige Gruppe ausgewählt aus der Gruppe bestehend aus CH₂, NH und O;
R^{a} und R^{b} gleich oder verschieden, ein Rest ausgewählt aus der Gruppe bestehend aus Wasserstoff, C₁₋₄-Alkyl, und Halogen-C₁₋₄-alkyl, oder
R^{a} und R^{b} gemeinsam eine C₂₋₅-alkylen-Brücke in der ein oder mehrere Wasserstoffatome gegebenenfalls ersetzt sein können durch Halogen;
R¹ und R^{1'} gleich oder verschieden, ein Rest ausgewählt aus der Gruppe bestehend aus Wasserstoff, C₁₋₆-Alkyl, C₃₋₆-Cyloalkyl, Halogen-C₁₋₆-alkyl, Halogen-C₃₋₆-cycloalkyl oder C₁₋₆-Alkylen-C₃₋₆-cycloalkyl, oder
R¹ und R^{1'} gemeinsam eine C₂₋₅-alkylen-Brücke in der ein oder mehrere Wasserstoffatome gegebenenfalls ersetzt sein können durch Halogen;;
R², R^{2'}, R^{2"} und R^{2"'} gleich oder verschieden, ein Rest ausgewählt aus der Gruppe bestehend aus Wasserstoff, C₁₋₆-Alkyl, Halogen-C₁₋₆-alkylen, OH, HO-C₁₋₆-alkylen, -O-C₁₋₆-Alkyl, C₆₋₁₀-Aryl, C₆₋₁₀-Aryl-C₁₋₄-alkylen, C₆₋₁₀-Aryl-C₁₋₆-alkylen-O-, COOH, COOC₁₋₆-alkyl, O-C ₁₋₆-alkylen-COOH, O-C₁₋₆-alkylen-COOC₁₋₆-alkyl, NHSO₂-C₁₋₆-alkyl, CN, NH₂, NH-C₁₋₆-Alkyl, N(C₁₋₆-Alkyl)₂, NO₂, S-C₁₋₆-Alkyl, SO₂-C₁₋₆-Alkyl, SO-C₁₋₆-Alkyl, O(CO)C₁₋₆-Alkyl, COC₁₋₆-Alkyl, NHCOC₁₋₆-Alkyl oder Halogen;
n 0, 1 oder 2;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

2. Verbindungen der Formel 1 nach Anspruch 1, worin
X -O-, -CH₂-O-, -C(Me)₂-O- oder -CH=CH- bedeutet;
V eine zweibindige Gruppe ausgewählt aus der Gruppe bestehend aus CH₂, NH und O;
R^{a} und R^{b} gleich oder verschieden, ein Rest ausgewählt aus der Gruppe bestehend aus Wasserstoff, C₁₋₄-Alkyl und Fluor-C₁₋₄-alkyl, oder
R^{a} und R^{b} gemeinsam eine Gruppe ausgewählt aus -CH₂-CH₂-, -CH₂-CH₂-CH₂-CH₂- und -CH₂-CH₂-CH₂-CH₂-CH_{z}-, in der ein oder mehrere Wasserstoffatome gegebenenfalls ersetzt sein können durch Fluor oder Chlor, bevorzugt Fluor;
R¹ und R^{1'} gleich oder verschieden, ein Rest ausgewählt aus der Gruppe bestehend aus Wasserstoff, C₁₋₆-Alkyl, C₃₋₆-Cycloalkyl, Halogen-C₁₋₆-alkyl oder C₁₋₆-Alkylen-C₃₋₆-Cycloalkyl, oder
R¹ und R^{1'} gemeinsam eine Gruppe ausgewählt aus -CH₂-CH₂-, -CH₂-CH₂-CH₂-CH₂- und -CH₂-CH₂-CH₂-CH₂-CH₂-, in der ein oder mehrere Wasserstoffatome gegebenenfalls ersetzt sein können durch Fluor oder Chlor, bevorzugt Fluor;
R², R^{2'}, R^{2''} und R^{2'''} gleich oder verschieden, ein Rest ausgewählt aus der Gruppe bestehend aus Wasserstoff, C₁₋₄-Alkyl, CF₃, CHF₂, CH₂F, OH, -O-C₁₋₄-Alkyl, Phenyl, Phenylethyl, Benzyl, Phenyloxy, Benzyloxy, COOH, COOC₁₋₄-alkyl, OCH₂COOH, OCH₂COOC₁₋₄-alkyl, NHSO₂-C₁₋₄-alkyl, Fluor, Chlor oder Brom;
n 0, 1 oder 2;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

3. Verbindungen der Formel 1 nach einem der Ansprüche 1 oder 2, worin
X -O-, -CH₂-O-, -C(Me)₂-O- oder -CH=CH-;
V eine zweibindige Gruppe ausgewählt aus der Gruppe bestehend aus CH₂ und O;
R^{a} und R^{b} gleich oder verschieden, ein Rest ausgewählt aus der Gruppe bestehend aus Wasserstoff, Methyl, Ethyl und CF₃, bevorzugt Wasserstoff, Methyl oder Ethyl, oder
R^{a} und R^{b} gemeinsam eine Gruppe ausgewählt aus -CH₂-CH₂- und -CH₂-CH₂-CH₂-CH₂-, bevorzugt -CH₂-CH₂-;
R¹ und R^{1'} gleich oder verschieden, ein Rest ausgewählt aus der Gruppe bestehend aus Wasserstoff, Methyl, Ethyl, Propyl, Cyclopropyl oder Methylcyclopropyl,
oder
R¹ und R^{1'} gemeinsam -CH₂-CH₂-CH₂-CH₂- oder -CH₂-CH₂-CH₂-CH₂-CH₂-;
R², R^{2'}, R^{2"} und R^{2"'} gleich oder verschieden, ein Rest ausgewählt aus der Gruppe bestehend aus Wasserstoff, Methyl, Ethyl, Propyl, CF₃, CHF₂, CH₂F, OH, Methyloxy, Ethyloxy, Propyloxy, COOH, COOCH₃, COOCH₂CH₃, OCH₂COOH, OCH₂COOCH₃, NHSO₂-CH₃, Fluor, Chlor oder Brom;
n 0, 1 oder 2;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

4. Verbindungen der Formel 1' 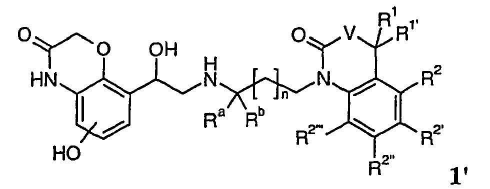 worin die Gruppen R^{a}, R^{b}, R¹, R^{1'}, R², R^{2'}, R^{2"}, R^{2"'} und V die in den Ansprüchen 1 bis 3 genannten Bedeutungen haben können.

5. Verbindungen der allgemeinen Formel 1' nach Anspruch 4, worin
V eine zweibindige Gruppe ausgewählt aus der Gruppe bestehend aus CH₂ und O;
R^{a} und R^{b} gleich oder verschieden, ein Rest ausgewählt aus der Gruppe bestehend aus Wasserstoff, C₁₋₄-Alkyl, und Halogen-C₁₋₄-alkyl, oder
R^{a} und R^{b} gemeinsam eine C₂₋₅-alkylen-Brücke in der ein oder mehrere Wasserstoffatome gegebenenfalls ersetzt sein können durch Halogen;
R¹ und R^{1'} gleich oder verschieden, ein Rest ausgewählt aus der Gruppe bestehend aus gleich oder verschieden Wasserstoff, Methyl, Ethyl, Propyl oder Cyclopropyl, oder
R¹ und R^{1'} gemeinsam -CH₂-CH₂-CH₂-CH₂- oder -CH₂-CH₂-CH₂-CH₂-CH₂-;
R² und R^{2'''} Wasserstoff;
R^{2'} und R^{2''} gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
n 0, 1 oder 2;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

6. Verbindungen der Formel **1"** 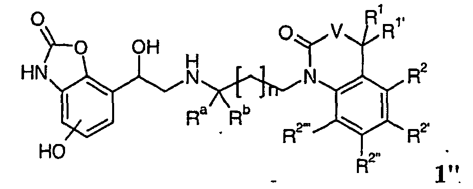 worin die Gruppen R^{a}, R^{b}, R¹, R^{1'}, R², R^{2'}, R^{2''}, R^{2'''} und V die in den Ansprüchen 1 bis 3 genannten Bedeutungen haben können.

7. Verbindungen der allgemeinen Formel 1' nach Anspruch 4, worin
V eine zweibindige Gruppe ausgewählt aus der Gruppe bestehend aus CH₂ und O;
R^{a} und R^{b} gleich oder verschieden, ein Rest ausgewählt aus der Gruppe bestehend aus Wasserstoff, C₁₋₄-Alkyl, und Halogen-C₁₋₄-alkyl, oder
R^{a} und R^{b} gemeinsam eine C₂₋₅-alkylen-Brücke in der ein oder mehrere Wasserstoffatome gegebenenfalls ersetzt sein können durch Halogen;
R¹ und R^{1'} gleich oder verschieden, ein Rest ausgewählt aus der Gruppe bestehend aus Wasserstoff, Methyl, Ethyl, Propyl, Cyclopropyl, oder
R¹ und R^{1'} gemeinsam -CH₂-CH₂-CH₂-CH₂- oder -CH₂-CH₂-CH₂-CH₂-CH₂-;
R² und R^{2'"} Wasserstoff;
R^{2'} und R^{2"} gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
n 0, 1 oder 2;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

8. Verbindungen der Formel **1**''' 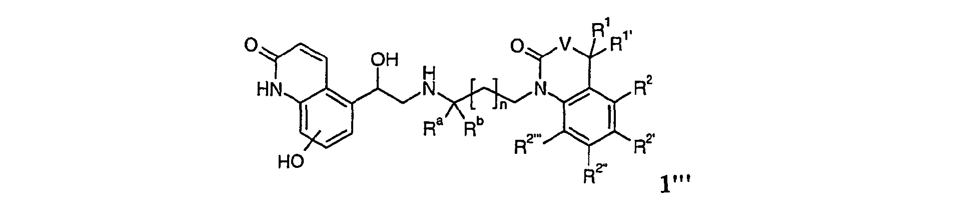 worin die Gruppen R^{a}, R^{b}, R¹, R^{1'}, R², R^{2'}, R^{2"}, R^{2'"} und V die in denAnsprüchen 1 bis 3 genannten Bedeutungen haben können.

9. Verbindungen der Formel 1"' nach Anspruch 8, worin
V eine zweibindige Gruppe ausgewählt aus der Gruppe bestehend aus CH₂ und O;
R^{a} und R^{b} gleich oder verschieden, ein Rest ausgewählt aus der Gruppe bestehend aus Wasserstoff, C₁₋₄-Alkyl, und Halogen-C₁₋₄-alkyl, oder
R^{a} und R^{b} gemeinsam eine C₂₋₅-alkylen-Brücke in der ein oder mehrere Wasserstoffatome gegebenenfalls ersetzt sein können durch Halogen;
R¹ und R^{1'} gleich oder verschieden, ein Rest ausgewählt aus der Gruppe bestehend aus Wasserstoff, Methyl, Ethyl, Propyl, Cyclopropyl, bevorzugt Methyl oder Ethyl,
oder
R¹ und R^{1'} gemeinsam -CH₂-CH₂-, -CH₂-CH₂-CH₂-CH₂- oder -CH₂-CH₂-CH₂-CH₂-CH₂-;
R² und R^{2"'} Wasserstoff;
R^{2'} und R^{2"} gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
n 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

10. Verbindungen der Formel 1'''' 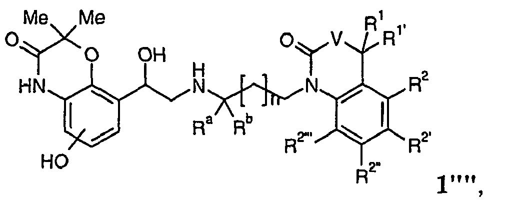 worin die Gruppen R^{a}, R^{b}, R¹, R¹', R², R^{2'}, R^{2"}, R^{2'"} und V die in denAnsprüchen 1 bis 3 genannten Bedeutungen haben können.

11. Verbindungen der Formel 1"" nach Anspruch 10, worin
V eine zweibindige Gruppe ausgewählt aus der Gruppe bestehend aus CH₂ und O;
R^{a} und R^{b} gleich oder verschieden, ein Rest ausgewählt aus der Gruppe bestehend aus Wasserstoff, C₁₋₄-Alkyl, und Halogen-C₁₋₄-alkyl, oder
R^{a} und R^{b} gemeinsam eine C₂₋₅-alkylen-Brücke in der ein oder mehrere Wasserstoffatome gegebenenfalls ersetzt sein können durch Halogen;
R¹ und R^{1'} gleich oder verschieden, ein Rest ausgewählt aus der Gruppe bestehend aus Wasserstoff, Methyl, Ethyl, Propyl, Cyclopropyl, oder
R¹ und R^{1'} gemeinsam -CH₂-CH₂-CH₂-CH₂- oder -CH₂-CH₂-CH₂-CH₂-CH₂-;
R² und R^{2"'} Wasserstoff;
R^{2'} und R^{2"} gleich oder verschieden, ein Rest ausgewählt aus der Gruppe bestehend aus Wasserstoff, Methyl, CF₃, OH, Methyloxy, Benzyloxy, COOH, COOCH₃ oder Fluor;
n 0, 1 oder 2, bevorzugt 1;
bedeuten, gegebenenfalls in Form der einzelnen optischen Isomeren, Mischungen der einzelnen Enantiomeren oder Racemate, in Form der freien Basen oder der entsprechenden Säureadditionssalze mit pharmakologisch unbedenklichen Säuren.

12. Verwendung der Verbindungen der Formel **1** gemäß einem der Ansprüche 1 bis 11 als Arzneimittel.

13. Verwendung der Verbindungen der Formel **1** gemäß einem der Ansprüche 1 bis 11 zur Herstellung eines Arzneimittels zur Behandlung von Erkrankungen, in denen therapeutisch wirksame Dosen eines Betamimetikums einen therapeutischen Nutzen entfalten können.

14. Pharmazeutische Formulierungen **gekennzeichnet durch** den Gehalt an einer oder mehreren Verbindungen der Formel **1** gemäß einem der Ansprüche 1 bis 11.

15. Inhalativ applizierbare pharmazeutische Formulierung, **gekennzeichnet durch** einen Gehalt einer Verbindung der Formel **1** gemäß einem der Ansprüche 1 bis 11.

16. Inhalativ applizierbare pharmazeutische Formulierung nach Anspruch 15, **dadurch gekennzeichnet, dass** sie ausgewählt ist aus der Gruppe bestehend aus Inhalationspulvern, treibgashaltige Dosieraerosole und treibgasfreie Inhalationslösungen.

## Claims

1. Compounds of formula 1 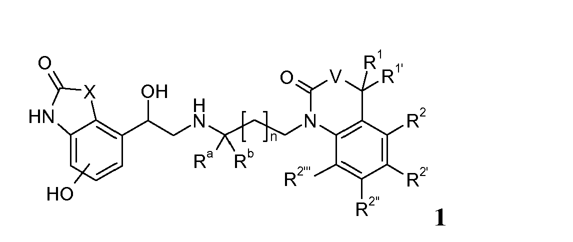 wherein
X denotes a group -O, -NH, -CH₂-O, -CHMe-O, -C(Me)₂-O, -CH₂-NH, -CHMe-NH, -C(Me)₂-NH, -CH=CH- or -CH₂-CH₂- ;
V denotes a double-bonded group selected from among CH₂, NH and O;
R^{a} and R^{b} which may be identical or different denote a group selected from among hydrogen, C₁₋₄-alkyl and halogen-C₁₋₄-alkyl, or
R^{a} and R^{b} together denote a C₂₋₅-alkylene bridge wherein one or more hydrogen atoms may optionally be replaced by halogen;
R¹ and R^{1'} which may be identical or different denote a group selected from among hydrogen, C₁₋₆-alkyl, C₃₋₆-cycloalkyl, halogen-C₁_₆-alkyl, halogen-C₃₋₆-cycloalkyl or C₁_₆-alkylene-C₃_₆-cycloalkyl, or
R¹ and R^{1'} together denote a C₂₋₅-alkylene bridge wherein one or more hydrogen atoms may optionally be replaced by halogen;
R², R^{2'}, R^{2"} and R^{2"'} which may be identical or different denote a group selected from among hydrogen, C₁₋₆-alkyl, halogen-C₁₋₆-alkylene, OH, HO-C₁₋₆-alkylene, -O-C₁₋₆-alkyl, C₆₋₁₀-aryl, C₆₋₁₀-aryl-C₁₋₄-alkylene, C₆₋₁₀-aryl-C₁₋₆-alkylene-O, COOH, COOC₁₋₆-alkyl, O-C₁₋₆-alkylene-COOH, O-C₁₋₆-alkylene-COOC₁₋₆-alkyl, NHSO₂-C₁₋₆-alkyl, CN, NH₂, NH-C₁₋₆-alkyl, N(C₁₋₆-alkyl)₂, NO₂, S-C₁₋₆-alkyl, SO₂-C₁₋₆-alkyl, SO-C₁₋₆-alkyl, O(CO)C₁₋₆-alkyl, COC₁₋₆-alkyl, NHCOC₁₋₆-alkyl or halogen;
n denotes 0, 1 or 2;
optionally in the form of the individual optical isomers, mixtures of the individual enantiomers or racemates, in the form of the free bases or the corresponding acid addition salts with pharmacologically acceptable acids.

2. Compounds of formula **1** according to claim 1, wherein
X denotes -O, -CH₂-O, -C(Me)₂-O- or -CH=CH-;
V denotes a double-bonded group selected from among CH₂, NH and O;
R^{a} and R^{b} which may be identical or different denote a group selected from among hydrogen, C₁₋₄-alkyl and fluoro-C₁₋₄-alkyl,
or
R^{a} and R^{b} together denote a group selected from -CH₂-CH₂, -CH₂-CH₂-CH₂-CH₂- and -CH₂ CH₂ CH₂ CH₂ CH₂, wherein one or more hydrogen atoms may optionally be replaced by fluorine or chlorine, preferably fluorine;
R¹ and R^{1'} which may be identical or different denote a group selected from among hydrogen, C₁₋₆-alkyl, C₃₋₆-cycloalkyl, halogen-C₁₋₆-alkyl or C₁₋₆-alkylene-C₃₋₆-cycloalkyl,
or
R¹ and R^{1'} together denote a group selected from -CH₂-CH₂, -CH₂-CH₂-CH₂-CH₂- and -CH₂ CH₂ CH₂ CH₂ CH₂, wherein one or more hydrogen atoms may optionally be replaced by fluorine or chlorine, preferably fluorine;
R², R^{2'}, R^{2''} and R^{2'''} which may be identical or different denote a group selected from among hydrogen, C₁₋₄-alkyl, CF₃, CHF₂, CH₂F, OH, -O-C₁₋₄-alkyl, phenyl, phenylethyl, benzyl, phenyloxy, benzyloxy, COOH, COOC₁₋₄-alkyl, OCH₂COOH, OCH₂COOC₁₋₄-alkyl, NHSO₂-C₁₋₄-alkyl, fluorine, chlorine or bromine;
n denotes 0, 1 or 2;
optionally in the form of the individual optical isomers, mixtures of the individual enantiomers or racemates, in the form of the free bases or the corresponding acid addition salts with pharmacologically acceptable acids.

3. Compounds of formula 1 according to one of claims 1 or 2, wherein
X denotes -O, -CH₂-O, -C(Me)₂-O- or -CH=CH-;
V denotes a double-bonded group selected from among CH₂ and O;
R^{a} and R^{b} which may be identical or different denote a group selected from among hydrogen, methyl, ethyl and CF₃, preferably hydrogen, methyl or ethyl, or
R^{a} and R^{b} together denote a group selected from -CH₂-CH₂- and -CH₂-CH₂-CH₂-CH₂, preferably -CH₂-CH₂-;
R¹ and R^{1'} which may be identical or different denote a group selected from among hydrogen, methyl, ethyl, propyl, cyclopropyl or methylcyclopropyl,
or
R¹ and R^{1'} together denote -CH₂-CH₂-CH₂-CH₂- or -CH₂-CH₂-CH₂-CH₂-CH₂-;
R², R^{2'}, R^{2"} and R^{2"'} which may be identical or different denote a group selected from among hydrogen, methyl, ethyl, propyl, CF₃, CHF₂, CH₂F, OH, methyloxy, ethyloxy, propyloxy, COOH, COOCH₃, COOCH₂CH₃, OCH₂COOH, OCH₂COOCH₃, NHSO₂-CH₃, fluorine, chlorine or bromine;
n denotes 0, 1 or 2;
optionally in the form of the individual optical isomers, mixtures of the individual enantiomers or racemates, in the form of the free bases or the corresponding acid addition salts with pharmacologically acceptable acids.

4. Compounds of formula **1**' 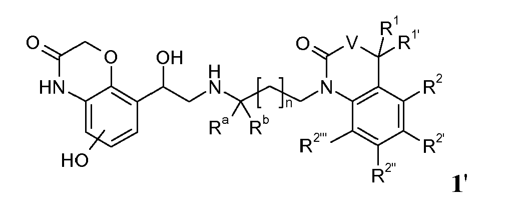 wherein the groups R^{a}, R^{b}, R¹, R^{1'}, R², R^{2'}, R^{2"}, R^{2'"} and V may have the meanings given in claims 1 to 3.

5. Compounds of general formula **1**' according to claim 4, wherein
V denotes a double-bonded group selected from among CH₂ and O;
R^{a} and R^{b} which may be identical or different denote a group selected from among hydrogen, C₁₋₄-alkyl, and halogen-C₁₋₄-alkyl,
or
R^{a} and R^{b} together denote a C₂₋₅-alkylene bridge wherein one or more hydrogen atoms may optionally be replaced by halogen;
R¹ and R^{1'} which may be identical or different denote a group selected from among hydrogen, methyl, ethyl, propyl or cyclopropyl, or
R¹ and R¹' together denote -CH₂-CH₂-CH₂-CH₂- or -CH₂-CH₂-CH₂-CH₂-CH₂-;
R² and R^{2"'} denote hydrogen;
R^{2'} and R^{2"} which may be identical or different denote a group selected from among hydrogen, methyl, CF₃, OH, methyloxy, benzyloxy, COOH, COOCH₃ or fluorine;
n denotes 0, 1 or 2;
optionally in the form of the individual optical isomers, mixtures of the individual enantiomers or racemates, in the form of the free bases or the corresponding acid addition salts with pharmacologically acceptable acids.

6. Compounds of formula **1**" 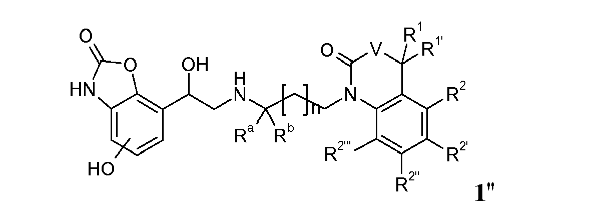 wherein the groups R^{a}, R^{b}, R¹, R^{1'}, R², R^{2'}, R^{2"}, R^{2'"} and V may have the meanings given in claims 1 to 3.

7. Compounds of general formula **1'** according to claim 4, wherein
V denotes a double-bonded group selected from among CH₂ and O;
R^{a} and R^{b} which may be identical or different denote a group selected from among hydrogen, C₁₋₄-alkyl, and halogen-C₁₋₄-alkyl, or
R^{a} and R^{b} together denote a C₂₋₅-alkylene bridge wherein one or more hydrogen atoms may optionally be replaced by halogen;
R¹ and R^{1'} which may be identical or different denote a group selected from among hydrogen, methyl, ethyl, propyl, cyclopropyl, or
R¹ and R^{1'} together denote -CH₂-CH₂-CH₂-CH₂- or -CH₂-CH₂-CH₂-CH₂-CH₂-;
R² and R^{2"'} denote hydrogen;
R^{2'} and R^{2"} which may be identical or different denote a group selected from among hydrogen, methyl, CF₃, OH, methyloxy, benzyloxy, COOH, COOCH₃ or fluorine;
n denotes 0, 1 or 2;
optionally in the form of the individual optical isomers, mixtures of the individual enantiomers or racemates, in the form of the free bases or the corresponding acid addition salts with pharmacologically acceptable acids.

8. Compounds of formula **1"'** 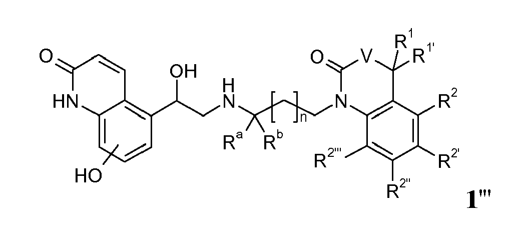 wherein the groups R^{a}, R^{b}, R¹, R^{1'}, R², R^{2'}, R^{2"}, R^{2"'} and V may have the meanings given in claims 1 to 3.

9. Compounds of formula **1"'** according to claim 8, wherein
V denotes a double-bonded group selected from among CH₂ and O;
R^{a} and R^{b} which may be identical or different denote a group selected from among hydrogen, C₁₋₄-alkyl and halogen-C₁₋₄-alkyl,
or
R^{a} and R^{b} together denote a C₂₋₅-alkylene bridge wherein one or more hydrogen atoms may optionally be replaced by halogen;
R¹ and R^{1'} which may be identical or different denote a group selected from among hydrogen, methyl, ethyl, propyl, cyclopropyl, preferably methyl or ethyl,
or
R¹ and R^{1'} together denote -CH₂-CH₂, -CH₂-CH₂-CH₂-CH₂- or -CH₂-CH₂-CH₂-CH₂-CH₂-;
R² and R^{2'"} denotes hydrogen;
R²' and R^{2"} which may be identical or different denote a group selected from among hydrogen, methyl, CF₃, OH, methyloxy, benzyloxy, COOH, COOCH₃ or fluorine;
n denotes 0, 1 or 2, preferably 1;
optionally in the form of the individual optical isomers, mixtures of the individual enantiomers or racemates, in the form of the free bases or the corresponding acid addition salts with pharmacologically acceptable acids.

10. Compounds of formula **1**"" 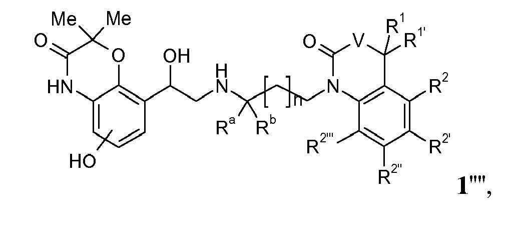 wherein the groups R^{a}, R^{b}, R¹, R^{1'}, R², R^{2'}, R^{2''}, R^{2'''} and V may have the meanings given in claims 1 to 3.

11. Compounds of formula **1**"" according to claim 10, wherein
V denotes a double-bonded group selected from among CH₂ and O;
R^{a} and R^{b} which may be identical or different denote a group selected from among hydrogen, C₁₋₄-alkyl and halogen-C₁₋₄-alkyl,
or
R^{a} and R^{b} together denote a C₂₋₅-alkylene bridge wherein one or more hydrogen atoms may optionally be replaced by halogen;
R¹ and R^{1'} which may be identical or different denote a group selected from among hydrogen, methyl, ethyl, propyl, cyclopropyl, or
R¹ and R^{1'} together denote -CH₂-CH₂-CH₂-CH₂- or -CH₂-CH₂-CH₂-CH₂-CH₂-;
R² and R^{2"'} denote hydrogen;
R^{2'} and R^{2"} which may be identical or different denote a group selected from among hydrogen, methyl, CF₃, OH, methyloxy, benzyloxy, COOH, COOCH₃ or fluorine;
n denotes 0, 1 or 2, preferably 1;
optionally in the form of the individual optical isomers, mixtures of the individual enantiomers or racemates, in the form of the free bases or the corresponding acid addition salts with pharmacologically acceptable acids.

12. Use of the compounds of formula 1 according to one of claims 1 to 11 as pharmaceutical compositions.

13. Use of the compounds of formula **1** according to one of claims 1 to 11 for preparing a pharmaceutical composition for the treatment of diseases in which therapeutically effective doses of a betamimetic may develop a therapeutic benefit.

14. Pharmaceutical formulations, **characterised in that** they contain one or more compounds of formula **1** according to one of claims 1 to 11.

15. Pharmaceutical formulation capable of being administered by inhalation, **characterised in that** it contains a compound of formula **1** according to one of claims 1 to 11.

16. Pharmaceutical formulation capable of being administered by inhalation according to claim 15, **characterised in that** it is selected from among the inhalable powders, propellant-containing metered-dose aerosols and propellant-free inhalable solutions.

## Revendications

1. Composés de formule 1 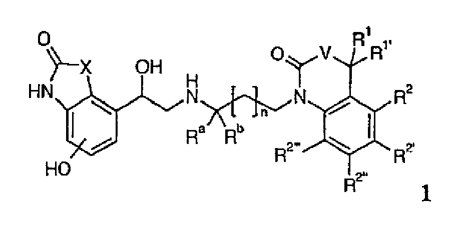 où
X représente un groupe -O-, -NH-, -CH₂-O-, -CHMe-O-, -C(Me)₂-O-, -CH₂-NH-, -CHMe-NH-, -C(Me)₂-NH-, -CH=CH- ou -CH₂-CH₂- ;
V représente un groupe divalent choisi dans le groupe consistant en CH₂, NH et O;
R^{a} et R^{b}, identiques ou différents, représentent un groupement choisi dans le groupe consistant en l'hydrogène, C₁₋₄-alkyle, et halogène-C₁₋₄-alkyle,
ou
R^{a} et R^{b} représentent ensemble un pont C₂₋₅-alkylène dans lequel un ou plusieurs atomes d'hydrogène peuvent éventuellement être remplacés par halogène ;
R¹ et R^{1'}, identiques ou différents, représentent un groupement choisi dans le groupe consistant en l'hydrogène, C₁₋₆-alkyle, C₃₋₆-cycloalkyle, halogène-C₁₋₆-alkyle, halogène-C₃₋₆-cycloalkyle ou C₁₋₆-alkylène-C₃₋₆-cycloalkyle, ou
R¹ et R^{1'} représentent ensemble un pont C₂₋₅-alkylène dans lequel un ou plusieurs atomes d'hydrogène peuvent éventuellement être remplacés par halogène ;
R², R^{2'}, R^{2"} et R^{2"'}, identiques ou différents, représentent un groupement choisi dans le groupe consistant en l'hydrogène, C₁₋₆-alkyle, halogène-C₁₋₆-alkylène, OH, HO-C₁₋₆-alkylène, -O-C₁₋₆-alkyle, C₆₋₁₀-aryle, C₆₋₁₀-aryl-C₁₋₄-alkylène, C₆₋₁₀-aryl- C₁-₄-alkylène-O-, COOH, COOC₁₋₆-alkyle, O-C₁₋₆-alkylène-COOH, O-C₁₋₆-alkylène-COOC₁₋₆-alkyle, NHSO₂-C₁₋₆-alkyle, CN, NH₂, NH-C₁₋₆-alkyle, N(C₁₋₆-alkyle)₂, NO₂, S-C₁₋₆-alkyle, SO₂-C₁₋₆-alkyle, SO-C₁₋₆-alkyle, O(CO)C₁₋₆-alkyle, COC₁₋₆-alkyle, NHCOC₁₋₆-alkyle ou halogène ;
n représente 0, 1 ou 2 ;
éventuellement sous forme des différents isomères optiques, de mélanges des différents énantiomères ou de racémates, sous forme des bases libres ou des sels d'addition d'acide correspondants avec des acides pharmacologiquement acceptables.

2. Composés de formule 1 selon la revendication 1, où
X représente -O-, -CH₂-O-, -C(Me)₂-O- ou -CH=CH- ;
V représente un groupe divalent choisi dans le groupe consistant en CH₂, NH et O;
R^{a} et R^{b}, identiques ou différents, représentent un groupement choisi dans le groupe consistant en l'hydrogène, C₁₋₄-alkyle et fluor-C₁₋₄-alkyle, ou
R^{a} et R^{b} représentent ensemble un groupe choisi parmi -CH₂-CH₂-, -CH₂-CH₂-CH₂-CH₂- et -CH₂-CH₂-CH₂-CH₂-CH₂- dans lequel un ou plusieurs atomes d'hydrogène peuvent éventuellement être remplacés par le fluor ou le chlore, de préférence le fluor ;
R¹ et R^{1'} identiques ou différents, représentent un groupement choisi dans le groupe consistant en l'hydrogène, C₁₋₆-akyle, C₃₋₆-cycloalkyle, halogène-C₁₋₆-alkyle ou C₁₋₆-akylène-C₃₋₆-cycloakyle,
ou
R¹ et R^{1'} représentent ensemble un groupe choisi parmi -CH₂-CH₂-, -CH₂-CH₂-CH₂-CH₂- et -CH₂-CH₂-CH₂-CH₂-CH₂- dans lequel un ou plusieurs atomes d'hydrogène peuvent éventuellement être remplacés par le fluor ou le chlore, de préférence le fluor ;
R², R^{2'}, R^{2''} et R^{2'''}, identiques ou différents, représentent un groupement choisi dans le groupe consistant en l'hydrogène, C₁₋₄-akyle, CF₃, CHF₂, CH₂F, OH, -OC₁₋₄-alkyle, phényle, phényléthyle, benzyle, phényloxy, benzyloxy, COOH, COOC₁₋₄-alkyle, OCH₂COOH, OCH₂COOC₁₋₄-alkyle, NHSO₂-C₁₋₄-alkyle, le fluor, le chlore ou le brome ;
n représente 0, 1 ou 2 ;
éventuellement sous forme des différents isomères optiques, de mélanges des différents énantiomères ou de racémates, sous forme des bases libres ou des sels d'addition d'acide correspondants avec des acides pharmacologiquement acceptables.

3. Composés de formule 1 selon l'une des revendications 1 ou 2, où
X représente -O-, -CH₂-O-, -C(Me)₂-O- ou -CH=CH- ;
V représente un groupe divalent choisi dans le groupe consistant en CH₂ et O ;
R^{a} et R^{b}, identiques ou différents, représentent un groupement choisi dans le groupe consistant en l'hydrogène, méthyle, éthyle et CF₃, de préférence l'hydrogène, méthyle ou éthyle, ou
R^{a} et R^{b} représentent ensemble un groupe choisi parmi -CH₂-CH₂- et -CH₂-CH₂-CH₂-CH₂-, de préférence -CH₂-CH₂- ;
R¹ et R^{1,}, identiques ou différents, représentent un groupement choisi dans le groupe consistant en l'hydrogène, méthyle, éthyle, propyle, cyclopropyle ou méthylcyclopropyle,
ou
R¹ et R^{1'} représentent ensemble -CH₂-CH₂-CH₂-CH₂- ou -CH₂-CH₂-CH₂-CH₂-CH₂-;
R², R^{2'}, R^{2"} et R^{2"'}, identiques ou différents, représentent un groupement choisi dans le groupe consistant en l'hydrogène, méthyle, éthyle propyle, CF₃, CHF₂, CH₂F, OH, méthyloxy, éthyloxy, propyloxy, COOH, COOCH₃, COOCH₂CH₃, OCH₂COOH, OCH₂COOCH₃, NHSO₂-CH₃, le fluor, le chlore ou le brome ;
n représente 0, 1 ou 2 ;
éventuellement sous forme des différents isomères optiques, de mélanges des différents énantiomères ou de racémates, sous forme des bases libres ou des sels d'addition d'acide correspondants avec des acides pharmacologiquement acceptables.

4. Composés de formule **1'** 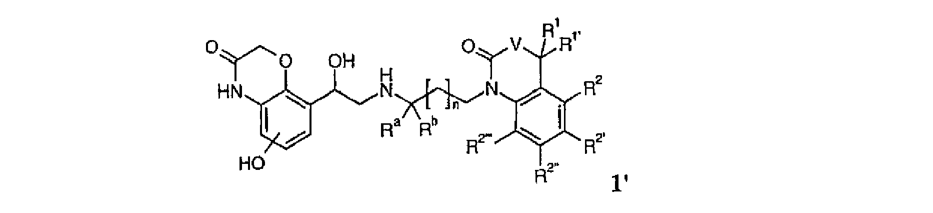 où les groupes R^{a}, R^{b}, R¹, R^{1'}, R², R^{2'}, R^{2"}, R^{2"'} et V peuvent avoir les significations citées dans les revendications 1 à 3.

5. Composés de formule générale **1'** selon la revendication **4** où
V représente un groupe divalent choisi dans le groupe consistant en CH₂ et O ;
R^{a} et R^{b}, identiques ou différents, représentent un groupement choisi dans le groupe consistant en l'hydrogène, C₁₋₄-alkyle, et halogène-C₁₋₄-alkyle,
ou
R^{a} et R^{b} représentent ensemble un pont C₂₋₅-alkylène dans lequel un ou plusieurs atomes d'hydrogène peuvent éventuellement être remplacés par halogène ;
R¹ et R^{1'}, identiques ou différents, représentent un groupement choisi dans le groupe consistant en l'hydrogène, méthyle, éthyle, propyle ou cyclopropyle identiques ou différents,
ou
R¹ et R^{1'} représentent ensemble -CH₂-CH₂-CH₂-CH₂- ou -CH₂-CH₂-CH₂-CH₂-CH₂- ;
R² et R^{2'''} représentent l'hydrogène ;
R^{2'} et R^{2''}, identiques ou différents, représentent un groupement choisi dans le groupe consistant en l'hydrogène, méthyle, CF₃, OH, méthyloxy, benzyloxy, COOH, COOCH₃ ou le fluor ;
n représente 0, 1 ou 2 ;
éventuellement sous forme des différents isomères optiques, de mélanges des différents énantiomères ou de racémates, sous forme des bases libres ou des sels d'addition d'acide correspondants avec des acides pharmacologiquement acceptables.

6. Composés de formule **1**" 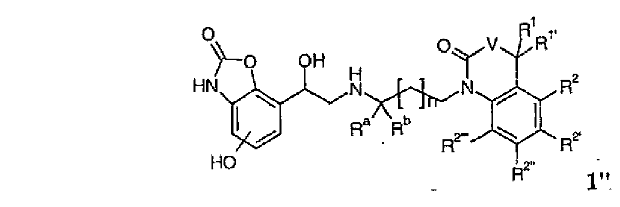 où les groupes R^{a}, R^{b}, R¹, R^{1'}, R², R^{2'}, R^{2"}, R^{2"'} et V peuvent avoir les significations citées dans les revendications 1 à 3.

7. Composés de formule générale **1**' selon la revendication 4 où
V représente un groupe divalent choisi dans le groupe consistant en CH₂ et O ;
R^{a} et R^{b}, identiques ou différents, représentent un groupement choisi dans le groupe consistant en l'hydrogène, C₁₋₄-alkyle et halogène-C₁₋₄-alkyle,
ou
R^{a} et R^{b} représentent ensemble un pont C₂₋₅-alkylène dans lequel un ou plusieurs atomes d'hydrogène peuvent éventuellement être remplacés par halogène ;
R¹ et R^{1'} identiques ou différents, représentent un groupement choisi dans le groupe consistant en l'hydrogène, méthyle, éthyle, propyle, cyclopropyle,
ou
R¹ et R^{1'} représentent ensemble -CH₂-CH₂-CH₂-CH₂- ou -CH₂-CH₂-CH₂-CH₂-CH₂- ;
R² et R^{2"'} représentent l'hydrogène ;
R^{2'} et R^{2"}, identiques ou différents, représentent un groupement choisi dans le groupe consistant en l'hydrogène, méthyle, CF₃, OH, méthyloxy, benzyloxy, COOH, COOCH₃ ou le fluor ;
n représente 0, 1 ou 2 ;
éventuellement sous forme des différents isomères optiques, de mélanges des différents énantiomères ou de racémates, sous forme des bases libres ou des sels d'addition d'acide correspondants avec des acides pharmacologiquement acceptables.

8. Composés de formule **1**"' 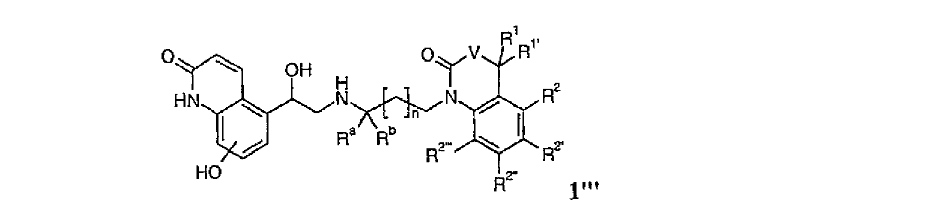 où les groupes R^{a}, R^{b}, R¹, R^{1'}, R², R^{2'}, R^{2''}, R^{2'''} et V peuvent avoir les significations citées dans les revendications 1 à 3.

9. Composés de formule générale **1**"' selon la revendication 8 où
V représente un groupe divalent choisi dans le groupe consistant en CH₂ et O ;
R^{a} et R^{b}, identiques ou différents, représentent un groupement choisi dans le groupe consistant en l'hydrogène, C₁₋₄-alkyle et halogène-C₁₋₄-alkyle,
ou
R^{a} et R^{b} représentent ensemble un pont C₂₋₅-alkylène dans lequel un ou plusieurs atomes d'hydrogène peuvent éventuellement être remplacés par halogène ;
R¹ et R^{1'} identiques ou différents, représentent un groupement choisi dans le groupe consistant en l'hydrogène, méthyle, éthyle, propyle, cyclopropyle, de préférence méthyle ou éthyle,
ou
R¹ et R^{1'} représentent ensemble -CH₂-CH₂-, -CH₂-CH₂-CH₂-CH₂- ou -CH₂-CH₂-CH₂-CH₂-CH₂- ;
R² et R^{2"'} représentent l'hydrogène ;
R^{2'} et R^{2"}, identiques ou différents, représentent un groupement choisi dans le groupe consistant en l'hydrogène, méthyle, CF₃, OH, méthyloxy, benzyloxy, COOH, COOCH₃ ou le fluor ;
n représente 0, 1 ou 2, de préférence 1 ;
éventuellement sous forme des différents isomères optiques, de mélanges des différents énantiomères ou de racémates, sous forme des bases libres ou des sels d'addition d'acide correspondants avec des acides pharmacologiquement acceptables.

10. Composés de formule **1**"" 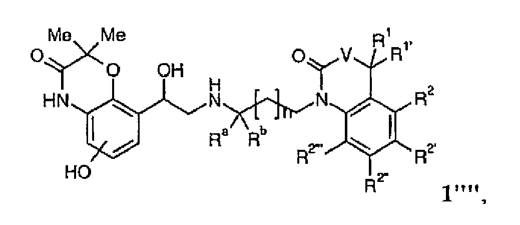 où les groupes R^{a}, R^{b}, R¹, R^{1'}, R², R^{2'}, R^{2''}, R^{2'''} et V peuvent avoir les significations citées dans les revendications 1 à 3.

11. Composés de formule générale **1**"" selon la revendication 10 où
V représente un groupe divalent choisi dans le groupe consistant en CH₂ et O ;
R^{a} et R^{b}, identiques ou différents, représentent un groupement choisi dans le groupe consistant en l'hydrogène, C₁₋₄-alkyle et halogène-C₁₋₄-alkyle,
ou
R^{a} et R^{b} représentent ensemble un pont C₂₋₅-alkylène dans lequel un ou plusieurs atomes d'hydrogène peuvent éventuellement être remplacés par halogène ;
R¹ et R^{1'}, identiques ou différents, représentent un groupement choisi dans le groupe consistant en l'hydrogène, méthyle, éthyle, propyle, cyclopropyle,
ou
R¹ et R^{1'} représentent ensemble -CH₂-CH₂-CH₂-CH₂- ou -CH₂-CH₂-CH₂-CH₂-CH₂- ;
R² et R^{2"'} représentent l'hydrogène ;
R^{2'} et R^{2"}, identiques ou différents, représentent un groupement choisi dans le groupe consistant en l'hydrogène, méthyle, CF₃, OH, méthyloxy, benzyloxy, COOH, COOCH₃ ou le fluor ;
n représente 0, 1 ou 2, de préférence 1 ;
éventuellement sous forme des différents isomères optiques, de mélanges des différents énantiomères ou de racémates, sous forme des bases libres ou des sels d'addition d'acide correspondants avec des acides pharmacologiquement acceptables.

12. Utilisation des composés de formule **1** selon l'une des revendications 1 à 11 comme médicament.

13. Utilisation des composés de formule **1** selon l'une des revendications 1 à 11 pour la production d'un médicament pour le traitement des maladies dans lesquelles des doses thérapeutiquement efficaces d'un bêtamimétique peuvent présenter une utilité thérapeutique.

14. Formulations pharmaceutiques **caractérisées par** la teneur en un ou plusieurs composés de formule **1** selon l'une des revendications 1 à 11.

15. Formulation pharmaceutique applicable par inhalation, **caractérisée par** une teneur d'un composé de formule **1** selon l'une des revendications 1 à 11.

16. Formulation pharmaceutique applicable par inhalation selon la revendication 15, **caractérisée en ce qu'**elle est choisie dans le groupe consistant en les poudres pour l'inhalation, les aérosols de dosage contenant un gaz propulseur et les solutions pour l'inhalation sans gaz propulseur.
